# EUROPÄISCHE PATENTANMELDUNG

(11) **EP 4 659 689 A1**
(43) Veröffentlichungstag der Anmeldung: **10.12.2025**
(21) Anmeldenummer: 25154492.0
(22) Anmeldetag: 28.01.2025
(51) Int. Cl.: A61B 17/14, A61B 17/16

(54) **RESEKTIONSVORRICHTUNG**

(30) Priorität: 03.06.2024 EP 24179522
(71) Anmelder: Medivation AG, 5400 Baden (CH)
(72) Erfinder: Stifter, Jan, 5425 Schneisingen (CH); Schmid, Patricia, 8051 Zürich (CH); Moor, Roman, 8006 Zürich (CH); Schwägli, Tobias, 4500 Solothurn (CH); Imboden, Gregor, 8045 Zürich (CH)
(74) Vertreter: Herrmann, Johanna

(57) **Zusammenfassung**

Eine Resektionsvorrichtung (10) zur Resektion mindestens eines Knochens einer Knochenanordnung (8) ohne Benutzerinteraktion umfasst ein Befestigungselement (4) zur starren Befestigung an der Knochenanordnung (8) sowie ein starr mit dem Befestigungselement (4) verbundenes Basiselement (5). Das Basiselement (5) enthält eine Basiselementlängsachse (15). Die Resektionsvorrichtung umfasst ferner ein drehbares Verbindungselement (1), welches drehbar um das Basiselement (5) angeordnet ist, wobei die Basiselementlängsachse (15) eine Drehachse (11) für das drehbare Verbindungselement (1) ausbildet, und ein verschiebbares Verbindungselement (2). Das verschiebbare Verbindungselement (2) führt eine Translation entlang der Drehachse (11) relativ zum Basiselement (5) aus. Die Resektionsvorrichtung (10) umfasst ferner ein Verbindungselement (3), welches über ein Drehgelenk (32) mit dem verschiebbaren Verbindungselement (2) oder dem drehbaren Verbindungselement (1) verbunden ist. Das Drehgelenk (32) enthält eine Drehgelenksdrehachse (31), sodass das Verbindungselement (3) um die Drehgelenksdrehachse (31) drehbar ist. Das Verbindungselement (3) enthält einen Endeffektor (6) zur Knochenresektion.

## Beschreibung

### Hintergrund

Die vorliegende Erfindung betrifft eine Resektionsvorrichtung zur Durchführung von Resektionseingriffen an Knochen. Die Resektionsvorrichtung ist dazu ausgebildet, an einer Knochenanordnung befestigt zu werden, um einen Resektionseingriff an zumindest einem der Knochen der Knochenanordnung durchzuführen. Die Resektionsvorrichtung umfasst insbesondere einen Endeffektor, an welchem ein Schneidwerkzeug oder Fräswerkzeug angebracht ist, welches dazu ausgebildet ist, während des Resektionseingriffs einer Vorgabe, beispielsweise einer vordefinierten Bahn zu folgen. Die Resektionsvorrichtung kann für robotergestützte chirurgische Eingriffe verwendet werden, insbesondere zur Erstellung präziser chirurgischer Resektionen von Knochen, beispielsweise zum Ersatz von Gelenken. Insbesondere kann die Resektionsvorrichtung für Knieoperationen eingesetzt werden, wenn ein Kniegelenk eines Patienten durch ein künstliches Kniegelenk ersetzt werden muss.

### Stand der Technik

Der Einsatz von Resektionsvorrichtungen erfolgt somit insbesondere in einem chirurgischen Eingriff, bei dem das Kniegelenk durch ein künstliches Kniegelenk ersetzt wird, dies wird auch als totaler Kniegelenkersatz (TKR) bezeichnet. Der Hauptgrund für diesen Eingriff ist Arthrose, andere Gründe sind rheumatoide Arthritis, posttraumatische Arthritis oder Gelenkverformungen. Allein in den USA wird dieser chirurgische Eingriff jährlich etwa 790.000-mal durchgeführt. Während des Eingriffs müssen präzise Schnitte am Oberschenkelknochen und am Schienbein durchgeführt werden. In der Regel werden fünf Schnitte am Oberschenkelknochen und ein Schnitt am Schienbein mit Hilfe von Schneidblöcken durchgeführt, die eine oszillierende Säge führen.

Um die Präzision dieser Schnitte zu erhöhen und ein Implantat genauer zu positionieren und den Eingriff für jeden Patienten leichter anpassbar zu machen, werden seit mehr als 20 Jahren Robotersysteme zur Unterstützung des Eingriffs eingesetzt. Beispielsweise enthalten Robotersysteme eine Schnittführungsvorrichtung, mittels welcher der Chirurg mit einer oszillierenden Säge schneiden kann. Robotersysteme können auch aus einem grossen Roboterarm mit handgehaltenem Endeffektor bestehen, mit dem der Chirurg den Schnitt durchführen kann, während er vom Robotersystem geführt wird, wie beispielsweise in US 11517380 B2 beschrieben. Diese Lösung erfordert ein Navigationssystem, welches die Position der Knochen sowie die Position des Endeffektors relativ zu den Knochen registrieren kann, also den gesamten Arbeitsbereich während des Eingriffs erfassen muss. Insbesondere kann sich der Chirurg innerhalb von einem vom Robotersystem vorgegebenen Bereich bewegen, wobei innerhalb dieses Bereichs keine Schnittführung erfolgt. Mit anderen Worten kann der Chirurg den Eingriff innerhalb dieses vorgegebenen Bereichs manuell ausführen.

Beispielsweise kann ein roboterunterstütztes System gemäss US 9 421 019 B2 zum Einsatz kommen, welches mittels einer Befestigungsvorrichtung am Knochen befestigt wird. Das Schneidwerkzeug wird danach mittels einer Einstelleinrichtung ausgerichtet. Das roboterunterstützte System muss allerdings für die Dauer des Eingriffs vom Chirurgen geführt und überwacht werden.

Bereits während der Planung des chirurgischen Eingriffs wird beispielsweise für einen totalen Kniegelenksersatz die Position des Implantats vorgegeben. Anhand der vorbestimmten Position werden die Schnitte definiert, die mit einer Resektionsvorrichtung durchgeführt werden müssen. Ein derartiges Verfahren ist beispielsweise im Dokument US 10 441 434 B2 beschrieben.

Falls für die Knochenresektion eine Fräsvorrichtung zum Einsatz kommt, muss ein Endeffektor der Fräsvorrichtung viele Anforderungen bezüglich Grösse, Steifigkeit, Drehzahl, Drehmoment, Lagerung erfüllen, wie beispielsweise im Dokument US 9 339 345 B2 beschrieben. Zudem erfordert der Einsatz einer Fräsvorrichtung für eine Knochenresektion einen geeigneten Fräspfad. Um diesen Fräspfad zu berechnen, gibt es verschiedene Strategien, dabei ist es essenziell, dass die Frässtrategie mit dem verwendeten System kompatibel ist. Im Dokument US 8 936 596 B2 wird eine Möglichkeit beschrieben, wie dies realisiert werden kann. Dabei werden ein Bild von einem vordefinierten Schnittmuster und ein Bild vom Knochen, welcher reseziert werden soll, übereinandergelegt. Anschliessend wird die Überschneidung vom Schnittmuster und dem Knochen berechnet, basierend darauf wird schliesslich ein Fräspfad berechnet. Bei der Berechnung wird darauf geachtet, dass der Schaden am Gewebe minimiert wird und dass jeweils ein dünner Rand entlang des Umfangs von der Schnittebene vom Knochen stehen bleibt.

Auch mit dem in US 20070123896 A1 beschriebenen chirurgischen Instrumentensystem wird die Knochenresektion von einem Chirurgen durchgeführt und die Positionierung des chirurgischen Instrumentensystems erfolgt manuell. Auch die im Dokument AU2017372744A1 gezeigte Resektionsvorrichtung wird von einem Chirurgen geführt, wofür Handgriffe vorgesehen sind, die für die manuelle Bedienung der Resektionsvorrichtung durch den Chirurgen bestimmt sind.

### Aufgabe der Erfindung

Aufgabe der Erfindung ist es, eine autonom operierende Resektionsvorrichtung bereitzustellen, mittels welcher es möglich ist, die Resektion ohne manuelle Unterstützung des Chirurgen und ohne den Einsatz eines computergestützten Navigationssystems, mit anderen Worten selbsttätig, auszuführen.

### Beschreibung der Erfindung

Die Lösung der Aufgabe der Erfindung erfolgt durch eine Resektionsvorrichtung gemäss Anspruch 1 oder Anspruch 3. Vorteilhafte Varianten der Resektionsvorrichtung sind Gegenstand der Ansprüche 2, bzw. 4 bis 15.

Wenn der Begriff "beispielsweise" in der nachfolgenden Beschreibung verwendet wird, bezieht sich dieser Begriff auf Ausführungsbeispiele und/oder Ausführungsformen, was nicht notwendigerweise als eine bevorzugtere Anwendung der Lehre der Erfindung zu verstehen ist. In ähnlicher Weise sind die Begriffe "vorzugsweise", "bevorzugt" zu verstehen, indem sie sich auf ein Beispiel aus einer Menge von Ausführungsbeispielen und/oder Ausführungsformen beziehen, was nicht notwendigerweise als eine bevorzugte Anwendung der Lehre der Erfindung zu verstehen ist. Dementsprechend können sich die Begriffe "beispielsweise", "vorzugsweise" oder "bevorzugt" auf eine Mehrzahl von Ausführungsbeispielen und/oder Ausführungsformen beziehen.

Die nachfolgende detaillierte Beschreibung enthält verschiedene Ausführungsbeispiele für die erfindungsgemässe Resektionsvorrichtung. Die Beschreibung einer bestimmten Resektionsvorrichtung ist nur als beispielhaft anzusehen. In der Beschreibung und den Ansprüchen werden die Begriffe "enthalten", "umfassen", "aufweisen" als "enthalten, aber nicht beschränkt auf" interpretiert.

Eine erfindungsgemässe Resektionsvorrichtung zur Resektion zumindest eines Knochens einer Knochenanordnung ohne Benutzerinteraktion umfasst ein Befestigungselement, wobei das Befestigungselement dazu ausgebildet ist, starr an der Knochenanordnung befestigt zu werden. Die Resektionsvorrichtung ist insbesondere als eine autonom arbeitende Resektionsvorrichtung ausgebildet. Unter einer autonom arbeitenden Resektionsvorrichtung wird eine Resektionsvorrichtung verstanden, die zumindest bei Durchführung der Resektion nicht vom Chirurgen geführt wird. Die Resektionsvorrichtung umfasst ferner ein Basiselement, wobei das Befestigungselement das Basiselement enthält oder das Befestigungselement mit dem Basiselement derart koppelbar ist, dass das Basiselement eine starre Verbindung mit dem Befestigungselement ausbildet. Das Basiselement enthält eine Basiselementlängsachse. Die Resektionsvorrichtung umfasst ferner ein drehbares Verbindungselement, welches drehbar um das Basiselement angeordnet ist, wobei das drehbare Verbindungselement drehbar um die Basiselementlängsachse angeordnet ist, sodass die Basiselementlängsachse eine Drehachse für das drehbare Verbindungselement ausbildet. Das drehbare Verbindungselement enthält einen ersten Verbindungselementantrieb.

Die Resektionsvorrichtung umfasst ferner ein verschiebbares Verbindungselement, wobei eine Linearbewegung des verschiebbaren Verbindungselements entlang der Drehachse relativ zum Basiselement erfolgen kann. Das verschiebbare Verbindungselement enthält einen zweiten Verbindungselementantrieb. Mittels des verschiebbaren Verbindungselements ist eine Linearbewegung entlang der Basiselementlängsachse relativ zum Basiselement ausführbar. Eine Linearbewegung wird als eine Translationsbewegung definiert, die in zwei zueinander entgegengesetzten Richtungen erfolgen kann. Die Resektionsvorrichtung umfasst ferner ein Verbindungselement, wobei das Verbindungselement über ein Drehgelenk mit einem der drehbaren oder verschiebbaren Verbindungselemente verbunden ist. Das Drehgelenk enthält eine Drehgelenksdrehachse, wobei das Verbindungselement um die Drehgelenksdrehachse drehbar ist. Das Verbindungselement enthält einen dritten Verbindungselementantrieb. Das Verbindungselement enthält einen Endeffektor zur Knochenresektion. Das Verbindungselement kann somit als eine Bearbeitungsvorrichtung zur Durchführung der Knochenresektion ausgebildet sein. Das Verbindungselement kann eine Einheit mit dem Endeffektor ausbilden. Der Endeffektor kann auch an das Verbindungselement gekoppelt werden. Beispielsweise können nach Bedarf unterschiedliche Endeffektoren am Verbindungselement angebracht werden.

Das Verbindungselement kann insbesondere als gelenkig koppelbares Verbindungselement ausgebildet sein. Der Begriff ohne Benutzerinteraktion bedeutet in diesem Zusammenhang, dass die Bearbeitung des Knochens durch die Resektionsvorrichtung autonom erfolgt.

Nach einem Ausführungsbeispiel ist die Drehgelenksdrehachse in einem Winkel von 60 Grad bis einschliesslich 90 Grad zur Drehachse angeordnet. Insbesondere kann die Drehgelenksdrehachse senkrecht zur Drehachse angeordnet sein.

Eine erfindungsgemässe Resektionsvorrichtung zur Resektion zumindest eines Knochens einer Knochenanordnung ohne Benutzerinteraktion umfasst auch ein Befestigungselement, wobei das Befestigungselement dazu ausgebildet ist, starr an der Knochenanordnung befestigt zu werden. Die Resektionsvorrichtung ist insbesondere als eine autonom arbeitende Resektionsvorrichtung ausgebildet. Unter einer autonom arbeitenden Resektionsvorrichtung wird eine Resektionsvorrichtung verstanden, die zumindest bei Durchführung der Resektion nicht vom Chirurgen geführt wird. Die Resektionsvorrichtung umfasst ferner ein Basiselement, wobei das Befestigungselement das Basiselement enthält oder das Befestigungselement mit dem Basiselement derart koppelbar ist, dass das Basiselement eine starre Verbindung mit dem Befestigungselement ausbildet. Das Basiselement enthält eine Basiselementlängsachse. Die Resektionsvorrichtung umfasst ferner ein drehbares Verbindungselement, welches drehbar um das Basiselement angeordnet ist, wobei das drehbare Verbindungselement drehbar um die Basiselementlängsachse angeordnet ist, sodass die Basiselementlängsachse eine Drehachse für das drehbare Verbindungselement ausbildet. Das drehbare Verbindungselement enthält einen ersten Verbindungselementantrieb.

Die Resektionsvorrichtung umfasst ferner ein verschiebbares Verbindungselement, wobei das verschiebbare Verbindungselement entlang der Drehachse eine Linearbewegung relativ zum Basiselement ausführen kann. Das verschiebbare Verbindungselement enthält einen zweiten Verbindungselementantrieb. Die Resektionsvorrichtung umfasst ferner ein Verbindungselement, wobei das Verbindungselement über ein Führungsschienenelement mit einem der drehbaren oder verschiebbaren Verbindungselemente verbunden ist. Das Führungsschienenelement enthält eine Schienenelementachse, wobei das Verbindungselement entlang der Schienenelementachse verschiebbar ist. Das Verbindungselement enthält einen Endeffektor zur Knochenresektion. Das Verbindungselement kann somit als eine Bearbeitungsvorrichtung zur Durchführung der Knochenresektion ausgebildet sein. Das Verbindungselement enthält einen dritten Verbindungselementantrieb. Das Verbindungselement kann eine Einheit mit dem Endeffektor ausbilden. Der Endeffektor kann auch an das Verbindungselement gekoppelt werden. Beispielsweise können nach Bedarf unterschiedliche Endeffektoren am Verbindungselement angebracht werden.

Nach einem Ausführungsbeispiel ist die Schienenelementachse in einem Winkel von 60 Grad bis einschliesslich 90 Grad zur Drehachse angeordnet. Insbesondere kann die Schienenelementachse senkrecht zur Drehachse angeordnet sein.

Nach einem Ausführungsbeispiel umfasst das Basiselement ein Hülsenelement und ein Kernelement. Insbesondere kann das Basiselement ein Eingriffselement enthalten, welches zur Aufnahme eines korrespondierenden Eingriffselements des drehbaren Verbindungselements ausgebildet ist. Nach einem Ausführungsbeispiel kann das Eingriffselement am Hülsenelement angebracht sein. Nach einem Ausführungsbeispiel ist das Eingriffselement als Abtriebszahnrad ausgebildet. Insbesondere kann das Abtriebszahnrad fest mit dem Hülsenelement verbunden sein.

Nach einem Ausführungsbeispiel enthält das drehbare Verbindungselement das korrespondierende Eingriffselement sowie den ersten Verbindungselementantrieb. Mittels des ersten Verbindungselementantriebs ist insbesondere das korrespondierende Eingriffselement antreibbar. Insbesondere kann das korrespondierende Eingriffselement als Antriebszahnrad ausgebildet sein. Insbesondere kann der erste Verbindungselementantrieb eine Antriebswelle und einen Antriebsmotor umfassen. Nach einem Ausführungsbeispiel enthält das drehbare Verbindungselement ein Gehäuse. Das Gehäuse ist insbesondere derart ausgebildet, dass es bei einer Betätigung des ersten Verbindungselementantriebs eine Drehung um die Drehachse des Basiselements ausführt. Insbesondere ist das Gehäuse um das Hülsenelement drehbar, wenn nach einem Ausführungsbeispiel ein derartiges Hülsenelement vorgesehen ist. Nach einem Ausführungsbeispiel umfasst das Gehäuse ein Gehäusemantelelement, ein Gehäusebodenelement und ein Gehäuseabdeckelement.

Nach einem Ausführungsbeispiel enthält der erste Verbindungselementantrieb ein Antriebsgehäuse für den Antriebsmotor. Das Antriebsgehäuse kann ebenfalls mit dem Gehäuse verbunden sein. Insbesondere kann das Antriebsgehäuse eine Drehbewegung des Gehäuses bei Betätigung des ersten Verbindungselementantriebs mitmachen. Nach einem Ausführungsbeispiel ist die Antriebswelle mittels des Antriebswellenlagerelements drehbar im Antriebsgehäuse gelagert.

Insbesondere kann bei Inbetriebnahme des Antriebsmotors die Antriebswelle in eine Drehbewegung versetzt werden. Die Antriebswelle ist insbesondere derart ausgebildet, dass sie das Antriebszahnrad in Bewegung versetzt. Das Antriebszahnrad ist insbesondere drehfest mit der Antriebswelle verbunden. Antriebswelle und Antriebszahnrad können auch ein einem einzigen Stück gefertigt sein. Nach einem Ausführungsbeispiel ist das Antriebszahnrad mit dem Abtriebszahnrad im Eingriff. Das Abtriebszahnrad ist insbesondere fest mit dem Hülsenelement verbunden. Das Antriebszahnrad kann nach der Art eines Planetengetriebes eine Umlaufbewegung um das Abtriebszahnrad ausführen. Diese Umlaufbewegung kann auf das Gehäuse übertragen werden. Nach einem Ausführungsbeispiel ist das Gehäuse somit mit dem ersten Verbindungselementantrieb um die Drehachse drehbar. Nach einem Ausführungsbeispiel enthält das Gehäuse das verschiebbare Verbindungselement.

Nach einem Ausführungsbeispiel ist das verschiebbare Verbindungselement über das Gehäuse mit dem drehbaren Verbindungselement gekoppelt. Nach diesem Ausführungsbeispiel ist das Gehäuse somit mit dem ersten Verbindungselementantrieb um die Drehachse drehbar, wobei das verschiebbare Verbindungselement diese Drehbewegung ebenfalls ausführt.

Die Drehung des drehbaren Verbindungselements kann im Uhrzeigersinn oder gegen den Uhrzeigersinn erfolgen. Die Drehrichtung des drehbaren Verbindungselements kann geändert werden. Insbesondere kann die Drehung der Antriebswelle im Uhrzeigersinn oder gegen den Uhrzeigersinn erfolgen, wenn der Antriebsmotor in beiden Drehrichtungen betrieben werden kann. Wenn die Resektionsvorrichtung für ein Fräsverfahren eingesetzt wird, muss es möglich sein, mittels der Resektionsvorrichtung einem bestimmten Pfad zu folgen. Für diese Verwendung muss eine Drehung der Antriebswelle in beide Richtungen möglich sein. Insbesondere ist jeder der drei Antriebsmotoren ausgebildet, um in zwei unterschiedlichen Drehrichtungen betrieben zu werden. Insbesondere kann die Drehrichtung jedes der Antriebsmotoren im Sekundentakt geändert werden, um dem Pfad zu folgen. Ein Motorencontroller kann zur Ansteuerung und zur Vorgabe der Drehrichtung vorgesehen sein.

Nach einem Ausführungsbeispiel ist das Befestigungselement zur Befestigung an einer Mehrzahl von Knochen der Knochenanordnung, insbesondere mindestens zwei benachbarten Knochen der Knochenanordnung ausgebildet, wobei das Befestigungselement ein Verbindungsstück zur Ausbildung einer starren Verbindung enthalten kann. Die starre Verbindung ermöglicht eine präzise Resektion des Knochens der Knochenanordnung oder jedes der Kochen der Knochenanordnung. Eine Knochenanordnung kann nach einem Ausführungsbeispiel auch aus nur einem einzigen Knochen bestehen. Insbesondere kann das Verbindungsstück mittels eines Befestigungsmittels am Befestigungselement oder an der Knochenanordnung befestigbar sein. Nach einem Ausführungsbeispiel ist das Verbindungsstück mittels entsprechender Befestigungsmittel an zwei Knochen der Knochenanordnung befestigt. Das Befestigungselement kann sich auch an einer anderen Stelle an der Knochenanordnung befinden als das Verbindungsstück. Das Befestigungselement kann auch nicht mit dem Verbindungsstück verbunden sein. Das Befestigungsmittel kann zumindest ein Befestigungselement aus der Gruppe bestehend aus Stiftelementen, Schraubelementen oder Klammerelementen umfassen.

Ein Schutzelement für Weichteile vor dem Endeffektor und vor externen Gegenständen, die den Arbeitsraum der Resektionsvorrichtung beeinträchtigen könnten, kann nach einem

Ausführungsbeispiel vorgesehen sein. Das Schutzelement kann beispielsweise als ein Schutzring oder Schutzbügel ausgebildet sein. Mittels des Schutzelements kann sichergestellt werden, dass bei der Montage oder im Betrieb des Endeffektors kein Gewebe oder andere Weichteile verletzt werden. Das Schutzelement kann insbesondere an einem Kopplungselement befestigt sein, welches mit dem Befestigungselement verbunden ist, wenn die Resektionsvorrichtung an der Knochenanordnung befestigt ist.

Nach einem Ausführungsbeispiel enthält das Befestigungselement mehrere Positionen zur Befestigung des Basiselements. Das Befestigungselement kann mehrteilig ausgebildet sein. Beispielsweise kann das Befestigungselement eine Mehrzahl von Befestigungsteilelementen umfassen. Jedes der Befestigungsteilelemente kann mindestens eine Position zur Befestigung des Basiselements aufweisen. Insbesondere können die Positionen des Befestigungselements mittels eines einstellbaren Kopplungselements einstellbar sein.

Nach einem Ausführungsbeispiel ist das Befestigungselement als ein intramedullärer Stab oder ein modifizierter intramedullärer Stab ausgebildet. Das Befestigungselement kann auch ein Befestigungsmittel aus der Gruppe, bestehend aus einer Knochenklammer, einem Knochennagel, einer Knochenschraube oder einer mit Stiftelementen befestigten Halterung enthalten.

Nach einem Ausführungsbeispiel ist das Werkzeug als ein Element aus der Gruppe bestehend aus einer Fräsvorrichtung, einer Sägevorrichtung oder einer Bohrvorrichtung, ausgebildet. Insbesondere kann das Werkzeug einen Kugelkopffräser enthalten.

Nach jedem der Ausführungsbeispiele können das drehbare Verbindungselement, das verschiebbare Verbindungselement und das Verbindungselement mittels entsprechenden Verbindungselementantrieben unabhängig voneinander antreibbar sein. Insbesondere kann das drehbare Verbindungselement durch einen ersten Verbindungselementantrieb angetrieben werden, das verschiebbare Verbindungselement durch einen zweiten Verbindungselementantrieb angetrieben werden und das Verbindungselement einen dritten Verbindungselementantrieb angetrieben werden. Insbesondere ist zumindest einer der Verbindungselementantriebe in einem kompakten, vom Gehäuse abnehmbaren Block untergebracht. Beispielsweise erleichtert die Anordnung von Motoren in einem kompakten, abnehmbaren Block deren Wartung und Montage.

Nach einem Ausführungsbeispiel sind der erste Verbindungselementantrieb und der zweite Verbindungselementantrieb in einem gemeinsamen Gehäuse angeordnet. Nach einem Ausführungsbeispiel wird mittels des ersten Verbindungselementantriebs eine Drehung des Gehäuses der Resektionsvorrichtung um die Basiselementlängsachse ausgeführt, wobei die Basiselementlängsachse die Drehachse ausbildet. Durch das Gehäuse wird nach diesem Ausführungsbeispiel das drehbare Verbindungselement ausgebildet. Das verschiebbare Verbindungselement ist nach diesem Ausführungsbeispiel so am Gehäuse angebracht, dass bei einer Drehung des drehbaren Verbindungselements das verschiebbare Verbindungselement von dieser Drehung mit erfasst wird.

Die Translationsbewegung des verschiebbaren Verbindungselements kann zeitgleich oder zeitlich versetzt in Bezug auf die Drehung des drehbaren Verbindungselements erfolgen. Es ist also möglich, dass das drehbare und das verschiebbare Verbindungselement gleichzeitig betätigt werden. Es ist auch möglich, dass zu einem ersten Zeitpunkt nur eine Drehung des drehbaren Verbindungselements erfolgt und zu einem zweiten Zeitpunkt nur die Verschiebung des verschiebbaren Verbindungselements erfolgt. Der erste Zeitpunkt kann sich somit vom zweiten Zeitpunkt unterscheiden. Der erste und der zweite Zeitpunkt können auch zusammenfallen, wenn der erste Verbindungselementantrieb und der zweite Verbindungselementantrieb gleichzeitig in Betrieb sind.

Auch die Drehung des Verbindungselements kann zeitgleich oder zeitlich versetzt in Bezug auf die Drehung drehbaren Verbindungselements oder die Translationsbewegung des verschiebbaren Verbindungselements erfolgen. Es ist auch möglich, dass zu einem ersten Zeitpunkt nur eine Drehung des drehbaren Verbindungselements erfolgt und zu einem zweiten Zeitpunkt nur die Verschiebung des verschiebbaren Verbindungselements erfolgt und zu einem dritten Zeitpunkt nur die Drehung oder Verschiebung des Verbindungselements erfolgt. Der erste Zeitpunkt kann sich somit von zumindest einem der zweiten oder dritten Zeitpunkte unterscheiden. Der erste, der zweite und der dritte Zeitpunkt können auch zusammenfallen, wenn der erste Verbindungselementantrieb und der zweite Verbindungselementantrieb und der dritte Verbindungselementantrieb gleichzeitig in Betrieb sind.

Das drehbare Verbindungselement, das verschiebbare Verbindungselement und das Verbindungselement können mittels entsprechenden Verbindungselementantrieben unabhängig voneinander antreibbar sein. Insbesondere kann das drehbare Verbindungselement durch einen ersten Verbindungselementantrieb angetrieben werden, das verschiebbare Verbindungselement durch einen zweiten Verbindungselementantrieb angetrieben werden und das Verbindungselement durch einen dritten Verbindungselementantrieb angetrieben werden. Insbesondere ist zumindest einer der Verbindungselementantriebe in einem kompakten, vom Gehäuse abnehmbaren Block untergebracht. Wenn durch das drehbare Verbindungselement das Gehäuse ausgebildet ist, kann das verschiebbare Verbindungselement am Gehäuse angeordnet sein. Beispielsweise erleichtert nach einem Ausführungsbeispiel die Anordnung des von zumindest zwei der ersten, zweiten oder dritten Verbindungselementantriebe in oder an einem kompakten, abnehmbaren Block die Wartung und Montage der Resektionsvorrichtung.

Nach einem Ausführungsbeispiel sind der erste Verbindungselementantrieb und der zweite Verbindungselementantrieb in oder an einem gemeinsamen Gehäuse angeordnet. Mittels des ersten Verbindungselementantriebs kann eine Drehung des Gehäuses der Resektionsvorrichtung um die Basiselementlängsachse ausgeführt werden, wobei die Basiselementlängsachse die Drehachse ausbildet. Durch das Gehäuse kann das drehbare Verbindungselement ausgebildet werden. Das verschiebbare Verbindungselement ist nach einem Ausführungsbeispiel so am Gehäuse angebracht, dass bei einer Drehung des drehbaren Verbindungselements das verschiebbare Verbindungselement von dieser Drehung mit erfasst wird.

Insbesondere weist die Resektionsvorrichtung nach jedem der Ausführungsbeispiele einen modularen Aufbau auf. Mittels des modularen Aufbaus ist ein Sterilkonzept einfacher realisierbar. Beispielsweise kann ein Teil der Resektionsvorrichtung steril ausgebildet sein, ein anderer Teil der Resektionsvorrichtung kann nicht steril ausgebildet sein. Zumindest ein Teil der Resektionsvorrichtung kann mit einer sterilen Abdeckung, die als eine sterile Hülle ausgebildet sein kann, abdeckbar sein.

Bei der Erfindung handelt es sich somit um eine robotergestützte Resektionsvorrichtung zur Durchführung aktiver, das heisst autonom durchgeführter, Resektionen von zumindest einem der Knochen der Knochenanordnung, z. B. des distalen Femurs bei einer Gelenkersatzoperation. Die Resektionsvorrichtung enthält ein Befestigungselement, ein Basiselement, welches starr mit dem Befestigungselement verbunden ist, und mehrere bewegliche Glieder, die als Verbindungselemente am Basiselement oder aneinander drehbar oder verschiebbar befestigt sind, wobei das letzte bewegliche Glied als ein Endeffektor ausgebildet ist. Der Endeffektor kann ein Knochenresektionswerkzeug enthalten, beispielsweise eine Fräsvorrichtung oder eine Sägevorrichtung. Die Befestigungsvorrichtung kann beispielsweise als zumindest ein Element aus der Gruppe bestehend aus einem intramedullären Stab, einem modifizierten intramedullären Stab, einem Knochennagel, einer Knochenschraube oder einer Knochenklammer ausgebildet sein.

Der Endeffektor folgt erfindungsgemäss autonom einem vordefinierten Pfad, ohne dass ein menschliches Eingreifen erforderlich ist, und reseziert den zumindest einen der Knochen der Knochenanordnung entsprechend. Die Resektion kann an dem Knochen der Knochenanordnung erfolgen, an dem die Resektionsvorrichtung angebracht ist, oder an einem benachbarten Knochen, z. B., wenn die Resektionsvorrichtung beispielsweise an einem intramedullären Stab im Femur angebracht wird und sowohl Femur als auch Tibia reseziert werden. Das Befestigungselement der Resektionsvorrichtung muss nicht zwingend neu positioniert werden, was eine nicht unbeträchtliche Verkürzung der Eingriffsdauer zur Folge haben kann. Die beiden Knochen der Knochenanordnung können durch ein Verbindungsstück starr miteinander verbunden sein.

Eine Anwendung für die erfindungsgemässe Resektionsvorrichtung kann die Resektion von Femur und Tibia für einen totalen Knieersatz sein.

Als weitere Vorteile der erfindungsgemässen Resektionsvorrichtung sind zu nennen, dass sie an der Knochenanordnung befestigt ist, ein geringes Eigengewicht aufweist, eine aktive, das heisst, autonome oder selbsttätige, Bearbeitung des zumindest einen Knochens der Knochenanordnung vornimmt. Durch die Befestigung der Resektionsvorrichtung an der Knochenanordnung kann die für den Eingriff benötigte Bearbeitungszeit reduziert werden, insbesondere weil das Setup sich einfacher gestalten lässt, insbesondere kann die Zeit bis zur Inbetriebnahme der Resektionsvorrichtung verkürzt werden, da eine Registrierung der Resektionsvorrichtung in Bezug auf den Patienten entfallen kann.

Die Resektionsvorrichtung wird erfindungsgemäss am Patienten in einer unveränderlichen Position befestigt. Weil der Schritt der Registrierung der Resektionsvorrichtung entfallen kann, kann die Bearbeitungszeit reduziert werden, das heisst, die Dauer des Eingriffs verkürzt werden, was eine geringere Belastung für den Patienten zur Folge hat. Mit der bei der Montage erfolgenden einmaligen Ausrichtung der Resektionsvorrichtung ist deren Position bereits zu Beginn des Eingriffs fixiert und kann sich durch die starre Fixierung nicht mehr verändern, sodass auch zusätzliche Arbeitszeit für nachträgliche Einstellungen am Patienten entfallen kann. Die Bauweise der erfindungsgemässen Resektionsvorrichtung ist besonders kompakt, da die Resektionsvorrichtung in ihrer Bauform auf die Knochenachse ausgerichtet ist. Aufgrund der kompakten Bauweise ist ein Navigationssystem nur für einen vergleichsweise kleinen Arbeitsbereich erforderlich. Zudem kann der kleine Arbeitsbereich präziser bearbeitet werden. Da die Resektionsvorrichtung eine kompaktere Bauweise als vorbekannte Lösungen aufweist, kann sie kostengünstiger hergestellt werden.

Wenn der Endeffektor zudem eine Fräsvorrichtung enthält, kann der Eingriff schonender erfolgen. Fräsen hat sich gemäss einer Vielzahl von Forschungsergebnissen als schonender für die Resektion des mindestens einen Knochens der Knochenanordnung erwiesen. Insbesondere entsteht durch den Fräsvorgang weniger Wärme. Durch die verminderte Hitzeeinwirkung entsteht weniger Schaden am Knochen. Die Resektionsvorrichtung arbeitet zudem mit erhöhter Präzision, weil sie direkt an der Knochenanordnung fixiert ist. Da die Resektionsvorrichtung ein einziges, kompaktes System ausbildet, welches starr mit dem Patienten verbunden bleibt, muss sie nicht vor dem Eingriff ausgerichtet werden. Im Gegensatz zu herkömmlichen, nicht mit dem Patienten verbundenen Robotersystemen, die ständig mit dem Patienten mitgeführt werden müssen, ist die erfindungsgemässe Resektionsvorrichtung bereits am Patienten fixiert. Somit kann der Eingriff am Patienten kostengünstiger erfolgen, es werden weniger Komponenten benötigt und die Handhabung während des Eingriffs gestaltet sich einfacher.

Zudem ist mit der erfindungsgemässen Resektionsvorrichtung eine Anordnung von drei Freiheitsgraden für den Betrieb des Endeffektors sowie eines mit dem Endeffektor gekoppelten Werkzeugs mittels der Resektionsvorrichtung selbst realisierbar, ohne dass es weiterer Bauelemente bedarf.

### Kurzbeschreibung der Zeichnungen

Nachfolgend werden die erfindungsgemässe Resektionsvorrichtung anhand eines Ausführungsbeispiels dargestellt. Es zeigen
Fig. 1 eine Seitenansicht einer Resektionsvorrichtung gemäss eines ersten Ausführungsbeispiels, die an einer Knochenanordnung befestigt ist,
Fig. 2 eine Seitenansicht der Resektionsvorrichtung gemäss Fig. 1,
Fig. 3 eine Frontalansicht der der Resektionsvorrichtung gemäss Fig. 1,
Fig. 4 eine perspektivische Ansicht der Resektionsvorrichtung gemäss Fig. 1,
Fig. 5 eine weitere perspektivische Ansicht der Resektionsvorrichtung gemäss Fig. 1,
Fig. 6 eine weitere perspektivische Ansicht der Resektionsvorrichtung gemäss Fig. 1,
Fig. 7 eine perspektivische Ansicht einer Variante der Resektionsvorrichtung gemäss Fig. 1,
Fig. 8 eine weitere perspektivische Ansicht der Resektionsvorrichtung gemäss Fig. 1,
Fig. 9 eine perspektivische Ansicht einer Variante der Resektionsvorrichtung gemäss Fig. 1,
Fig. 10 eine perspektivische Ansicht einer Resektionsvorrichtung und eines Verbindungsstücks gemäss einer ersten Variante zur Herstellung einer starren Verbindung zwischen Femur und Tibia,
Fig. 11 eine perspektivische Ansicht eines Verbindungsstücks gemäss einer zweiten Variante zur Herstellung einer starren Verbindung zwischen Femur und Tibia,
Fig. 12 eine perspektivische Ansicht eines Verbindungsstücks gemäss einer dritten Variante zur Herstellung einer starren Verbindung zwischen Femur und Tibia,
Fig. 13 eine perspektivische Ansicht eines Verbindungsstücks gemäss einer vierten Variante zur Herstellung einer starren Verbindung zwischen Femur und Tibia,
Fig. 14 eine perspektivische Ansicht eines Verbindungsstücks gemäss einer fünften Variante zur Herstellung einer starren Verbindung zwischen Femur und Tibia,
Fig. 15 eine Seitenansicht einer Resektionsvorrichtung gemäss eines zweiten Ausführungsbeispiels,
Fig. 16 eine Ansicht einer Resektionsvorrichtung mit einem Gewebeschutz,
Fig. 17 eine Ansicht einer Resektionsvorrichtung mit einer Variante des Befestigungselements,
Fig. 18 eine Ansicht einer Resektionsvorrichtung gemäss Fig. 1 mit teilweise weggelassenen Gehäuse.
Fig. 19a eine erste Ansicht einer Resektionsvorrichtung nach einem dritten Ausführungsbeispiel,
Fig. 19b einen Schnitt durch die Resektionsvorrichtung nach Fig. 19a entlang der Schnittebene B-B,
Fig. 20a eine zweite Ansicht einer Resektionsvorrichtung nach dem dritten Ausführungsbeispiel,
Fig. 20b einen Schnitt durch die Resektionsvorrichtung nach Fig. 20a entlang der Schnittebene C-C.

### Detaillierte Beschreibung

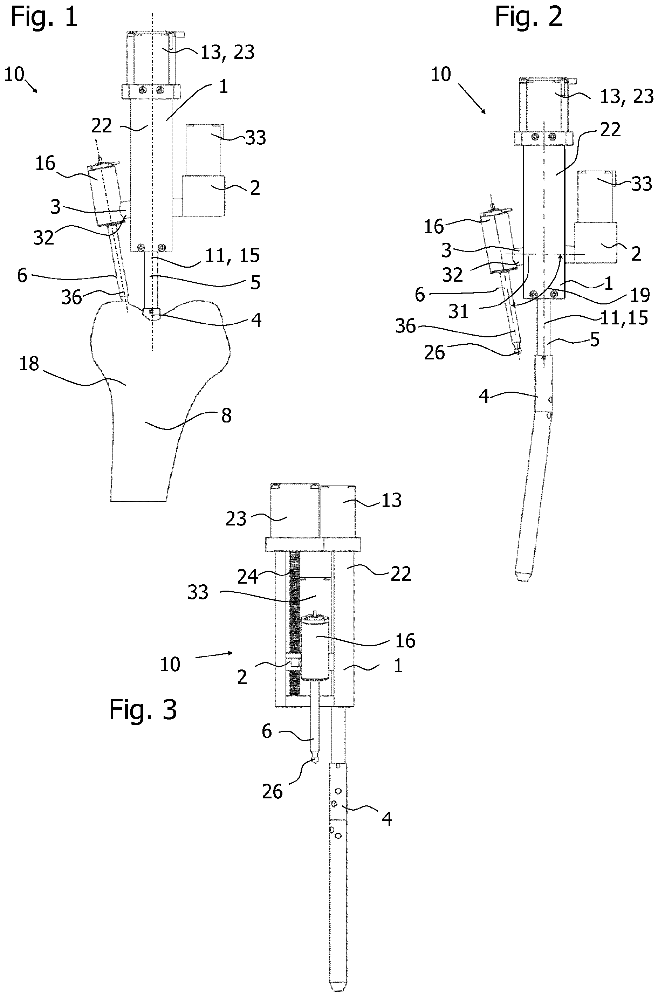

Fig. 1 zeigt eine Seitenansicht einer Resektionsvorrichtung 10 gemäss eines ersten Ausführungsbeispiels, die an einer Knochenanordnung 8 befestigt ist, Fig. 2 eine Seitenansicht der Resektionsvorrichtung 10 gemäss Fig. 1 und Fig. 3 eine Frontalansicht der der Resektionsvorrichtung gemäss Fig. 1. Die Resektionsvorrichtung 10 zur Resektion zumindest eines Knochens der Knochenanordnung 8 ohne Benutzerinteraktion umfasst ein Befestigungselement 4, wobei das Befestigungselement 4 dazu ausgebildet ist, starr an der Knochenanordnung 8 befestigt zu werden. Die Resektionsvorrichtung 10 umfasst ferner ein Basiselement 5, wobei das Befestigungselement das Basiselement 5 enthält oder das Befestigungselement 4 mit dem Basiselement 5 derart koppelbar ist, dass das Basiselement 5 eine starre Verbindung mit dem Befestigungselement 4 ausbildet. Das Basiselement 5 enthält eine Basiselementlängsachse 15. Die Resektionsvorrichtung 10 umfasst weiters ein drehbares Verbindungselement 1, welches drehbar um das Basiselement 5 angeordnet ist, wobei das drehbare Verbindungselement 1 drehbar um die Basiselementlängsachse 15 angeordnet ist, sodass die Basiselementlängsachse 15 eine Drehachse 11 für das drehbare Verbindungselement 1 ausbildet. Das drehbare Verbindungselement kann ein Gehäuse umfassen. Die Resektionsvorrichtung 10 umfasst weiters ein verschiebbares Verbindungselement 2, wobei das verschiebbare Verbindungselement 2 eine Linearbewegung entlang der Drehachse 11 relativ zum Basiselement 5 ausführen kann.

Die Resektionsvorrichtung 10 umfasst weiters ein Verbindungselement 3, wobei das Verbindungselement 3 über ein Drehgelenk 32 mit einem der drehbaren oder verschiebbaren Verbindungselemente 1,2 verbunden ist. Das Verbindungselement 3 ist als eine Bearbeitungsvorrichtung zur Durchführung der Knochenresektion ausgebildet. Das Drehgelenk 32 enthält eine Drehgelenksdrehachse 31, wobei das Verbindungselement 3 um die Drehgelenksdrehachse 31 drehbar ist, wobei die Drehgelenksdrehachse 31 nach diesem Ausführungsbeispiel senkrecht zur Drehachse 11 angeordnet ist. Das Verbindungselement 3 enthält einen Endeffektor 6 zur Knochenresektion. Das Verbindungselement 3 kann eine Einheit mit dem Endeffektor 6 ausbilden. Der Endeffektor 6 kann auch an das Verbindungselement 3 gekoppelt werden. Beispielsweise können nach Bedarf unterschiedliche Endeffektoren 6 am Verbindungselement 3 angebracht werden. Selbstverständlich kann nach einem nicht dargestellten Ausführungsbeispiel das mit dem Bezugszeichen 2 versehene Verbindungselement als das drehbare Verbindungselement ausgebildet sein und das mit dem Bezugszeichen 1 versehene Verbindungselement als das verschiebbare Verbindungselement ausgebildet sein.

Der Endeffektor 6 enthält das Werkzeug 26, insbesondere ein Knochenresektionswerkzeug, bei dem es sich beispielsweise um eine Fräsvorrichtung oder einen Sägeelement handeln kann. Der Endeffektor 6 kann eine Endeffektordrehachse 36 enthalten, um welche eine Rotation des Werkzeugs 26 erfolgen kann. Die Endeffektordrehachse 36 kann mit der Längsachse des Endeffektors 6 zusammenfallen. Zudem kann der Endeffektor einen Endeffektorantrieb 16 aufweisen.

Das drehbare Verbindungselement 1, das verschiebbare Verbindungselement 2 und das Verbindungselement 3 können mittels entsprechenden Verbindungselementantrieben 13, 23, 33 unabhängig voneinander antreibbar sein. Insbesondere kann das drehbare Verbindungselement 1 durch einen ersten Verbindungselementantrieb 13 angetrieben werden, das verschiebbare Verbindungselement 2 durch einen zweiten Verbindungselementantrieb 23 angetrieben werden und das Verbindungselement 3 durch einen dritten Verbindungselementantrieb 33 angetrieben werden. Insbesondere ist zumindest einer der Verbindungselementantriebe 13, 23, 33 in einem kompakten, vom Gehäuse abnehmbaren Block untergebracht. Nach dem vorliegenden Ausführungsbeispiel bildet das drehbare Verbindungselement 1 ein Gehäuse 22 aus und das verschiebbare Verbindungselement 2 ist am Gehäuse 22 angeordnet. Nach einem nicht dargestellten Ausführungsbeispiel wird vom drehbaren Verbindungselement 1 und vom verschiebbaren Verbindungselement 2 ein gemeinsames Gehäuse 22 ausgebildet.

Nach diesem Ausführungsbeispiel sind der erste Verbindungselementantrieb 13 und der zweite Verbindungselementantrieb 23 in oder an einem gemeinsamen Gehäuse 22 angeordnet. Mittels des ersten Verbindungselementantriebs 1 wird eine Drehung des Gehäuses 22 der Resektionsvorrichtung 10 um die Basiselementlängsachse 15 ausgeführt, wobei die Basiselementlängsachse 15 die Drehachse 11 ausbildet. Durch das Gehäuse 22 wird nach diesem Ausführungsbeispiel das drehbare Verbindungselement 1 ausgebildet. Das verschiebbare Verbindungselement 2 ist nach diesem Ausführungsbeispiel so am Gehäuse 22 angebracht, dass bei einer Drehung des drehbaren Verbindungselements 1 das verschiebbare Verbindungselement 2 von dieser Drehung mit erfasst wird. Die Translationsbewegung des verschiebbaren Verbindungselements 2 kann zeitgleich oder zeitlich versetzt in Bezug auf die Drehung des drehbaren Verbindungselements 1 erfolgen. Es ist also möglich, dass das drehbare und das verschiebbare Verbindungselement 1, 2 gleichzeitig betätigt werden.

Es ist auch möglich, dass zu einem ersten Zeitpunkt nur eine Drehung des drehbaren Verbindungselements 1 erfolgt und zu einem zweiten Zeitpunkt nur die Verschiebung des verschiebbaren Verbindungselements 2 erfolgt. Der erste Zeitpunkt kann sich somit vom zweiten Zeitpunkt unterscheiden. Der erste und der zweite Zeitpunkt können auch zusammenfallen, wenn der erste Verbindungselementantrieb 13 und der zweite Verbindungselementantrieb 23 gleichzeitig in Betrieb sind. Auch die Drehung des Verbindungselements 3 kann zeitgleich oder zeitlich versetzt in Bezug auf die Drehung drehbaren Verbindungselements 1 oder die Translationsbewegung des verschiebbaren Verbindungselements 2 erfolgen. Es ist auch möglich, dass zu einem ersten Zeitpunkt nur eine Drehung des drehbaren Verbindungselements 1 erfolgt und zu einem zweiten Zeitpunkt nur die Verschiebung des verschiebbaren Verbindungselements 2 erfolgt und zu einem dritten Zeitpunkt nur die Drehung oder Verschiebung des Verbindungselements 3 erfolgt. Der erste Zeitpunkt kann sich somit von zumindest einem der zweiten oder dritten Zeitpunkte unterscheiden. Der erste, der zweite und der dritte Zeitpunkt können auch zusammenfallen, wenn der erste Verbindungselementantrieb 13, der zweite Verbindungselementantrieb 23 und der dritte Verbindungselementantrieb 33 gleichzeitig in Betrieb sind.

Das Befestigungselement 4 kann beispielsweise als intramedullärer (IM) Stab oder als eine andere Fixierung ausgebildet sein. Die Fixierung kann an einer Knochenanordnung 8, zum Beispiel einem Femur 18 angebracht werden, wie in Fig. 1 gezeigt. Die Knochenanordnung 8 kann aus einem einzigen Knochen bestehen oder eine Mehrzahl von Knochen umfassen.

Gemäss dieses Ausführungsbeispiels verfügt die Resektionsvorrichtung über zumindest 3 Freiheitsgrade (DOF). Der erste DOF umfasst eine Rotation des drehbaren Verbindungselements 1 um die Drehachse 11, welche der Basiselementlängsachse 15 des Basiselements 5 entspricht oder nach einem nicht zeichnerisch dargestellten Ausführungsbeispiel parallel zu der Basiselementlängsachse 15 verläuft. Der zweite DOF umfasst eine Translationsbewegung des verschiebbaren Verbindungselements 2 in Richtung der Drehachse 11 des drehbaren Verbindungselements 1. Der dritte DOF umfasst nach dem vorliegenden Ausführungsbeispiel eine Rotation des Verbindungselements 3 um eine Drehgelenksdrehachse 31. Die Drehgelenksdrehachse kann insbesondere senkrecht zur Drehachse 11 des drehbaren Verbindungselements 1 stehen, sie kann beispielsweise auch in einem Winkel von 60 Grad bis einschliesslich 90 Grad zur Drehachse 11 angeordnet sein.

Fig. 2 zeigt eine Seitenansicht der Resektionsvorrichtung 10 gemäss Fig. 1. Gleiche oder gleichwirkende Bauelemente tragen dieselben Bezugszeichen wie in Fig. 1. Zu diesen Bauteilen wird auf die Beschreibung in Fig. 1 verwiesen. In analoger Weise wird auch für die gleichen oder gleichwirkenden Bauteile der Fig. 2-10 sowie 16-18 auf die Beschreibung zu Fig. 1 verwiesen.

In Fig. 2 ist als exemplarisches Ausführungsbeispiel gezeigt, dass die Endeffektordrehachse 36 des Endeffektors 6 in einem Neigungswinkel 19 zur Drehgelenksdrehachse 31 angeordnet ist. Wenn die Endeffektordrehachse 36 einen Neigungswinkel 19 mit der Drehgelenksdrehachse 31 einschliesst, kann mittels des Endeffektors 6 eine grössere Arbeitsfläche bearbeitet werden. Insbesondere können auch Bereiche der Arbeitsfläche bearbeitet werden, die sich an der Knochenanordnung 8 in der zeichnerischen Darstellung unterhalb des Gehäuses 22 befinden. Der Neigungswinkel 19 kann 60 bis einschliesslich 90 Grad betragen. Der in Fig. 2 dargestellte Neigungswinkel 19 ist nur als ein exemplarisches Ausführungsbeispiel für eine Vielzahl von möglichen Neigungswinkeln anzusehen.

Unterschiedliche Neigungswinkel können bei Bedarf durch unterschiedliche Bauarten des Verbindungselements 3 realisiert werden.

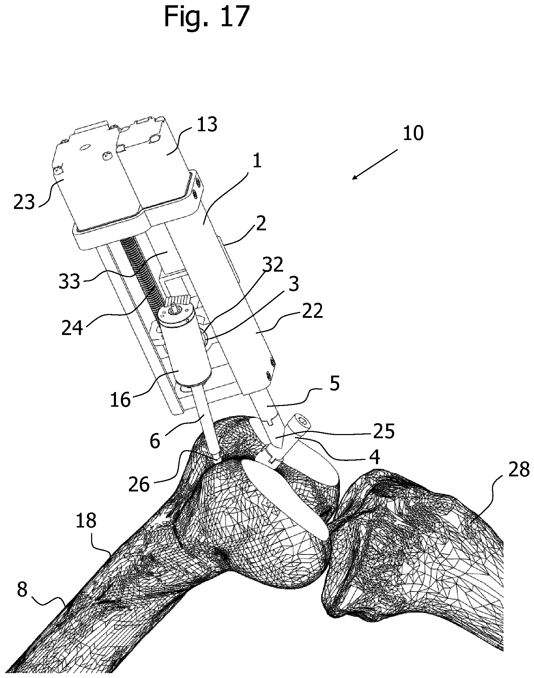

Insbesondere kann das Befestigungselement 4 mehrere Positionen zur Befestigung des Basiselements 5 enthalten, sodass die Resektionsvorrichtung 10 bereits bestmöglich in Bezug auf den Arbeitsbereich positioniert werden kann. Gegebenenfalls können die Positionen mittels eines einstellbaren Kopplungselements eingestellt werden, welches beispielsweise in Fig. 17 abgebildet ist.

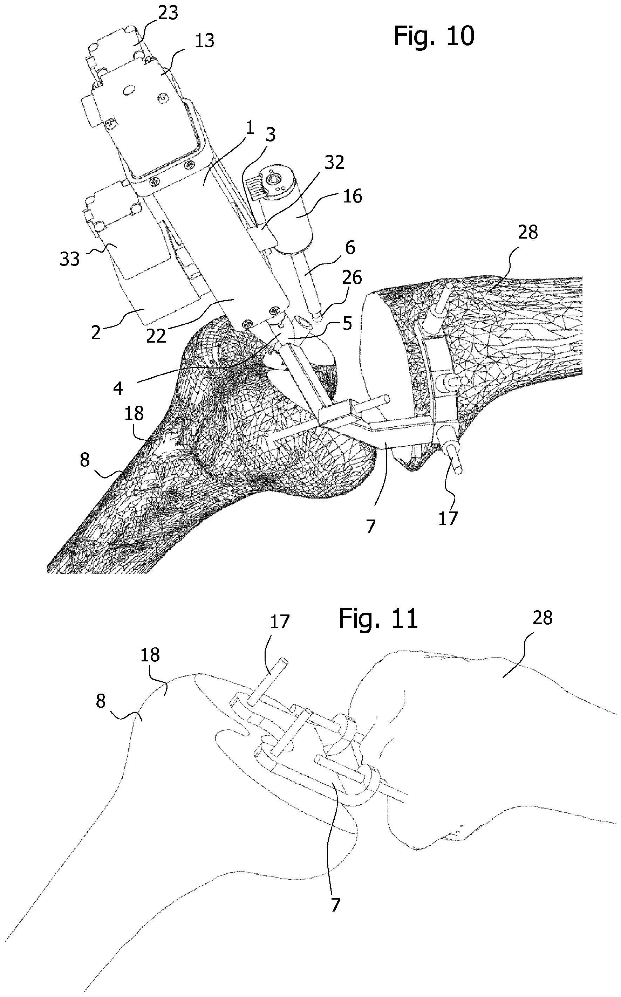

Das Befestigungselement 4 kann als ein Element aus der Gruppe bestehend aus einem intramedullären Stab, einem modifizierten intramedullären Stab, einem Knochennagel, einer Knochenschraube oder einer Knochenklammer ausgebildet sein. Das Befestigungselement 4 kann ein Verbindungsstück 7 enthalten oder mit einem Verbindungsstück 7 starr koppelbar sein. Ausführungsbeispiele für Befestigungselemente und verschiedene Varianten von Verbindungsstücken 7 sind in Fig. 10 bis 14 gezeigt.

Das Werkzeug 26 kann ein Element aus der Gruppe bestehend aus einer Fräsvorrichtung, einer Sägevorrichtung oder einer Bohrvorrichtung, umfassen. Insbesondere kann das Werkzeug 26 einen Kugelkopffräser enthalten.

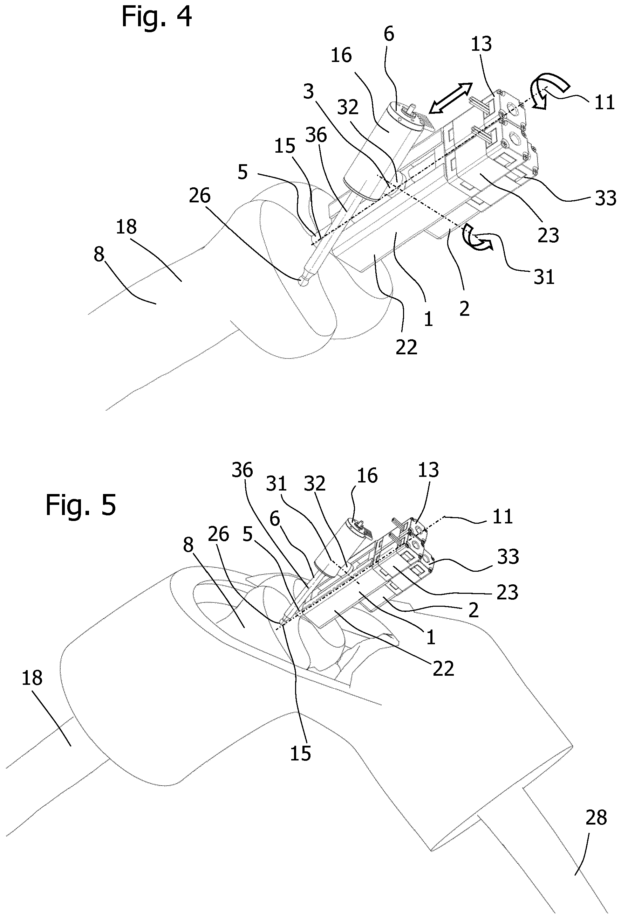

Fig. 4 zeigt eine perspektivische Ansicht der Resektionsvorrichtung 10 gemäss Fig. 1, die an einem Befestigungselement, welches als intramedullärer Stab ausgebildet ist, am Femur 18 befestigt ist. Die distale Resektion des Femurs 18 für einen totalen Knieersatz ist bereits erfolgt.

Fig. 5 zeigt eine perspektivische Ansicht der Resektionsvorrichtung 10 gemäss Fig. 1, die an einem Befestigungselement, welches als intramedullärer Stab ausgebildet ist, am Femur 18 befestigt ist, mit einer groben Modellierung des den Resektionsbereich umgebenden Weichteilgewebes. Die distale Resektion des Femurs 18 für einen totalen Knieersatz ist bereits erfolgt.

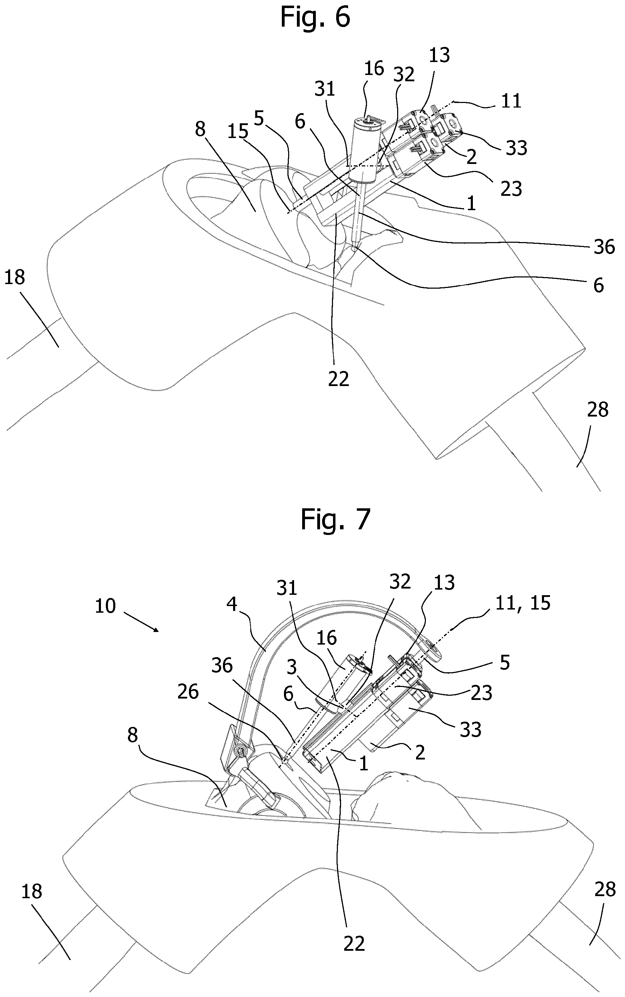

Fig. 6 zeigt eine perspektivische Ansicht der Resektionsvorrichtung 10 gemäss Fig. 1, die an einem Befestigungselement, welches als intramedullärer Stab ausgebildet ist, am Femur 18 befestigt ist, mit einer groben Modellierung des umgebenden Weichteilgewebes. Die distale Resektion des Femurs 18 für einen totalen Knieersatz ist bereits erfolgt, und die Resektionsvorrichtung 10 führt die Resektion der Tibia 28 durch.

Fig. 7 zeigt eine perspektivische Ansicht der Resektionsvorrichtung 10 gemäss Fig. 1 gemäss einer Variante. Das Befestigungselement 4 der Resektionsvorrichtung 10 gemäss Fig. 7 ist in Form einer speziellen Knochenklammer am Femur 18 ausgebildet. Fig. 7 zeigt auch eine grobe Modellierung des umgebenden Weichteilgewebes. Die distale Resektion des Femurs 18 für einen totalen Knieersatz ist bereits durchgeführt.

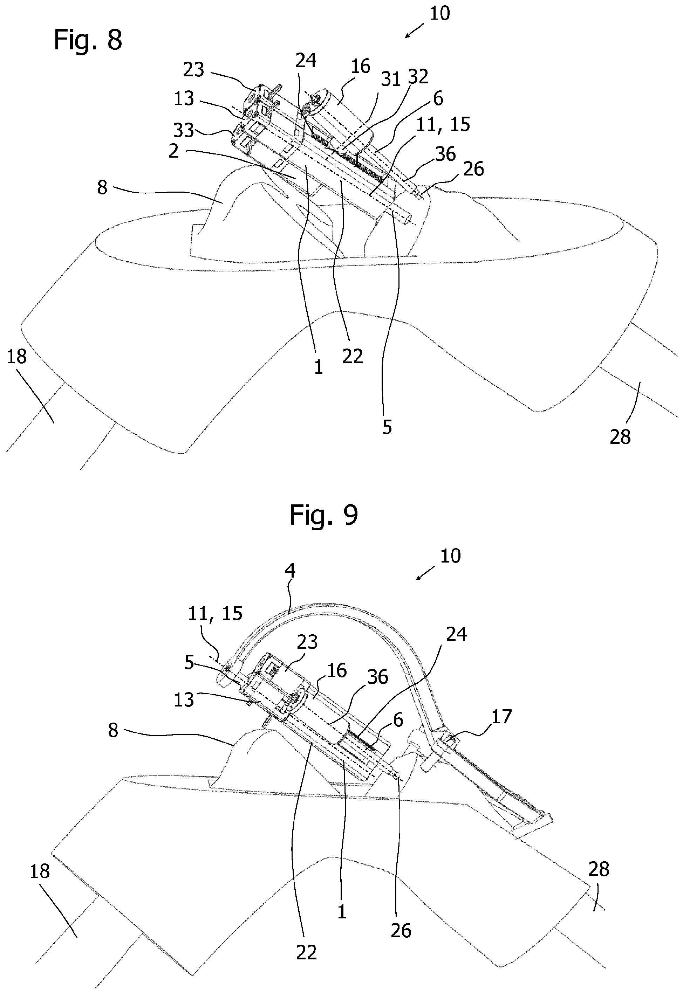

Fig. 8 zeigt eine perspektivische Ansicht der Resektionsvorrichtung 10 gemäss Fig. 1, die an einem Befestigungselement, welches als intramedullärer Stab ausgebildet ist, an der Tibia 28 befestigt ist, mit einer groben Modellierung des umgebenden Weichteilgewebes. Die distale Resektion des Femurs 18 und die proximale Resektion der Tibia 28 für einen totalen Knieersatz sind bereits durchgeführt.

Fig. 9 zeigt eine perspektivische Ansicht einer Variante der Resektionsvorrichtung 10 gemäss Fig. 1, das Befestigungselement 4 ist dabei in Form einer speziellen Halterung ausgebildet, welche mit Knochennägeln 17 an der Tibia 28 befestigt ist, mit einer groben Modellierung des umgebenden Weichteilgewebes. Die distale Resektion des Femurs 18 und die proximale Resektion der Tibia 28 für einen totalen Knieersatz sind bereits durchgeführt.

Fig. 10 zeigt eine perspektivische Ansicht der Resektionsvorrichtung 10 gemäss Fig. 1, des Femurs 18 und der Tibia 28 mit einer möglichen Konfiguration eines Befestigungselements 4 zur Herstellung einer starren Verbindung zwischen Femur 18 und Tibia 28. Das Befestigungselement 4 enthält ein Verbindungsstück 7 gemäss einer ersten Variante. Das Verbindungsstück 7 wird nach dem vorliegenden Ausführungsbeispiel am Befestigungselement 4 des Femurs 18 befestigt und mit Knochennägeln 17 an der Tibia 28 fixiert. Wenn die Resektionsvorrichtung 10 zur Resektion eines anderen Knochens der Knochenanordnung 8 verwendet wird, der nicht dem Knochen der Knochenanordnung 8 entspricht, an dem sie befestigt ist, z. B. zur Resektion der Tibia 28, während sie am Femur 18 befestigt ist. Wie in Fig. 6 gezeigt, werden Femur 18 und Tibia 28 mit Hilfe des Verbindungsstücks 7 starr miteinander verbunden. Bei dem Verbindungsstück 7 kann es sich um eine Platte mit Knochennägeln 17 zur Befestigung an der Knochenanordnung handeln, wie in Fig. 11 dargestellt. Andere Möglichkeiten sind die Verwendung von Knochenklammern 9 und Kombinationen aus Knochennägeln 17 und Knochenklammern 9 zur Befestigung des Verbindungsstücks 7 an der Knochenanordnung 8 sind in Fig. 11 bis Fig. 14 dargestellt. Anstelle von Knochennägeln können gemäss jedes der Ausführungsbeispiele auch Knochenstifte oder Knochenschrauben verwendet werden.

Fig. 11 zeigt eine perspektivische Ansicht des Femurs 18 und der Tibia 28 mit einer möglichen Konfiguration eines Verbindungsstücks 7 gemäss einer zweiten Variante zur Herstellung einer starren Verbindung zwischen Femur 18 und Tibia 28. Das Verbindungsstück 7 ist gemäss dieses Ausführungsbeispiels als eine Platte ausgebildet, die Knochennägeln 17 am distalen Ende des Femurs 18 und am proximalen Ende der Tibia 28 befestigt ist.

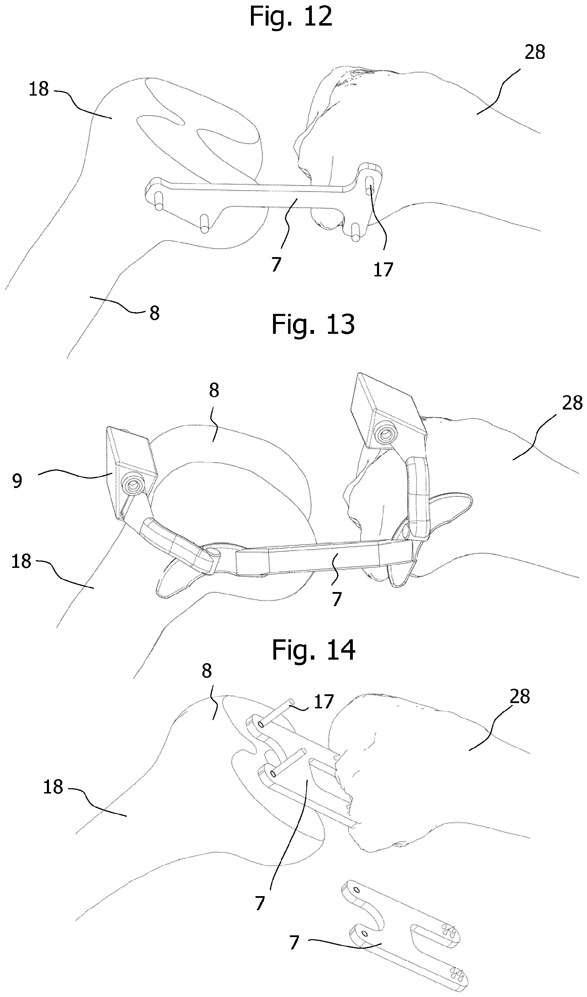

Fig. 12 zeigt eine perspektivische Ansicht des Femurs 18 und der Tibia 28 mit einer möglichen Konfiguration eines Verbindungsstücks 7 gemäss einer dritten Variante zur Herstellung einer starren Verbindung zwischen Femur 18 und Tibia 28. Das Verbindungsstück 7 wird mit Knochennägeln 17 von der medialen Seite im Femur und Tibia her befestigt.

Fig. 13 zeigt eine perspektivische Ansicht von Femur 18 und Tibia 28 mit einer möglichen Konfiguration eines Verbindungsstücks 7 gemäss einer vierten Variante zur Herstellung einer starren Verbindung zwischen Femur 18 und Tibia 28. Das Verbindungsstück wird mit Knochenklammern 9 auf Femur und Tibia direkt befestigt.

Fig. 14 zeigt eine perspektivische Ansicht von Femur 18 und Tibia 28 mit einer möglichen Konfiguration eines Verbindungsstücks 7 gemäss einer fünften Variante zur Herstellung einer starren Verbindung zwischen Femur 18 und Tibia 28. Das Verbindungsstück 7 nach diesem Ausführungsbeispiel ist eine Platte, die mit Knochennägeln 17 am distalen Ende des Femurs 18 und einer Knochenklammer am proximalen Ende der Tibia 28 befestigt ist.

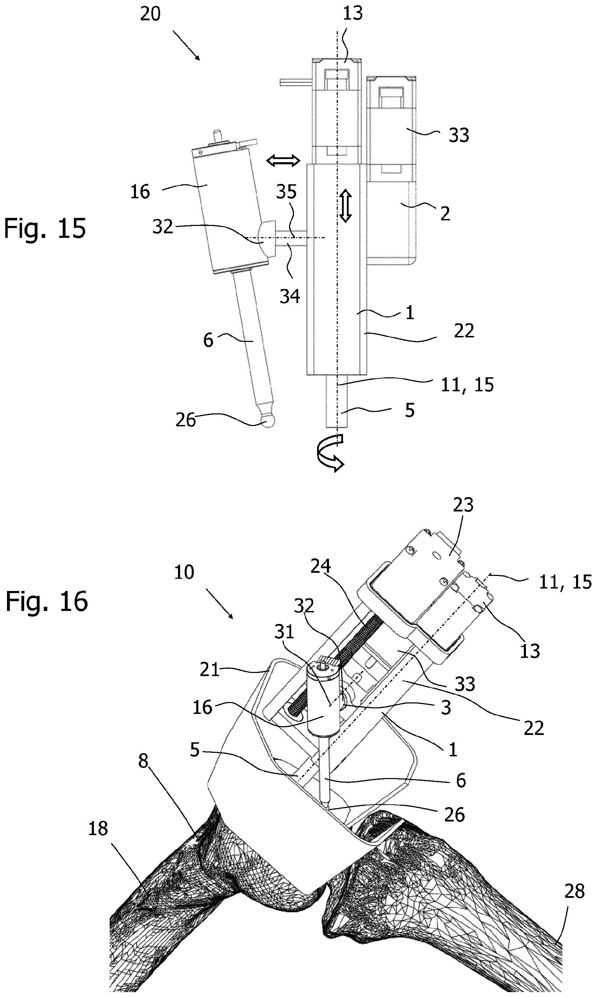

Fig. 15 zeigt eine Seitenansicht einer Resektionsvorrichtung 20 nach einem zweiten Ausführungsbeispiel. Nach dem vorliegenden Ausführungsbeispiel entsprechen die Bewegungsabläufe den ersten und zweiten Freiheitsgraden des ersten Ausführungsbeispiels. Für dieses Ausführungsbeispiel werden für gleiche oder gleichwirkende Elemente dieselben Bezugszeichen verwendet wie in den vorhergehenden Ausführungsbeispielen. Der dritte Freiheitsgrad umfasst nach diesem Ausführungsbeispiel eine Translation senkrecht zur Rotationsachse des ersten Freiheitsgrads.

Die Resektionsvorrichtung 20 zur Resektion zumindest eines Knochens einer Knochenanordnung 8 ohne Benutzerinteraktion nach dem in Fig. 15 gezeigten Ausführungsbeispiel enthält wie die in Fig. 1 dargestellte Resektionsvorrichtung 10 ein Befestigungselement 4, wobei das Befestigungselement 4 dazu ausgebildet ist, starr an der Knochenanordnung befestigt zu werden. Das Befestigungselement 4 ist in der vorliegenden Darstellung weggelassen, siehe hierzu insbesondere Fig. 1 bis Fig. 3. Zudem umfasst die Resektionsvorrichtung 20 ein Basiselement 5, wobei das Befestigungselement 4 das Basiselement 5 enthält oder das Befestigungselement 4 mit dem Basiselement 5 derart koppelbar ist, dass das Basiselement 5 eine starre Verbindung mit dem Befestigungselement 4 ausbildet. Das Basiselement 5 enthält eine Basiselementlängsachse 15. Die Resektionsvorrichtung 20 umfasst ein drehbares Verbindungselement 1, welches drehbar um das Basiselement 5 angeordnet ist, wobei das drehbare Verbindungselement 1 drehbar um die Basiselementlängsachse 15 angeordnet ist, sodass die Basiselementlängsachse 15 eine Drehachse 11 für das drehbare Verbindungselement 1 ausbildet.

Die Resektionsvorrichtung 20 umfasst ferner ein verschiebbares Verbindungselement 2, wobei das verschiebbare Verbindungselement 2 eine Linearbewegung entlang der Drehachse 11 relativ zum Basiselement 5 ausführen kann. Die Resektionsvorrichtung 20 umfasst zudem ein Verbindungselement 3, wobei das Verbindungselement 3 über ein Führungsschienenelement 34 mit dem verschiebbares Verbindungselement 2 verbunden ist. Das Führungsschienenelement 34 enthält eine Schienenelementachse 35, wobei das Verbindungselement 3 entlang der Schienenelementachse 35 verschiebbar ist. Die Schienenelementachse 35 ist nach diesem Ausführungsbeispiel senkrecht zur Drehachse 11 angeordnet. Das Verbindungselement 3 enthält einen Endeffektor 6 zur Knochen resektion.

Insbesondere kann das Befestigungselement 4 mehrere Positionen zur Befestigung des Basiselements 5 enthalten, sodass die Resektionsvorrichtung bereits bestmöglich in Bezug auf die Arbeitsfläche(n) positioniert werden kann. Gegebenenfalls können die Positionen mittels eines einstellbaren Kopplungselements eingestellt werden, welches beispielsweise in Fig. 17 abgebildet ist.

Das Befestigungselement 4 kann als ein Befestigungsmittel aus der Gruppe bestehend aus einem intramedullären Stab, einem modifizierten intramedullären Stab, einem Knochennagel, einer Knochenklammer, einer Knochenschraube oder einer mit Stiftelementen befestigten Halterung ausgebildet sein. Das Befestigungselement 4 kann ein Verbindungsstück 7 enthalten oder mit einem Verbindungsstück 7 starr koppelbar sein. Ausführungsbeispiele für Befestigungselemente und gegebenenfalls zugehörige Verbindungsstücke 7 sind in Fig. 10 bis 14 gezeigt.

Das Werkzeug 26 kann ein Element aus der Gruppe bestehend aus einer Fräsvorrichtung, einer Sägevorrichtung oder einer Bohrvorrichtung, umfassen. Insbesondere kann das Werkzeug 26 einen Kugelkopffräser enthalten.

Fig. 16 zeigt eine Ansicht einer Resektionsvorrichtung 10 mit einem Schutzelement 21 für Weichteile. Das Schutzelement 21 für Weichteile hat die Funktion, Weichteile vor dem Endeffektor 6 und/oder vor externen Gegenständen, die den Arbeitsraum der Resektionsvorrichtung beeinträchtigen können zu schützen. Das Schutzelement 21 kann insbesondere als ein Gewebeschutz ausgebildet sein. Das Schutzelement 21 kann an zumindest einer der Knochenanordnung 8, dem Befestigungselement 4, dem Basiselement 5 oder dem Verbindungsstück 7 befestigt werden.

Fig. 17 zeigt eine Ansicht einer Resektionsvorrichtung 10 enthaltend eine Variante des Befestigungselements 4, welches mehrere Positionen zur Befestigung des Basiselements 5 enthält. Gemäss des vorliegenden Ausführungsbeispiels sind die Positionen mittels eines einstellbaren Kopplungselements 25 einstellbar.

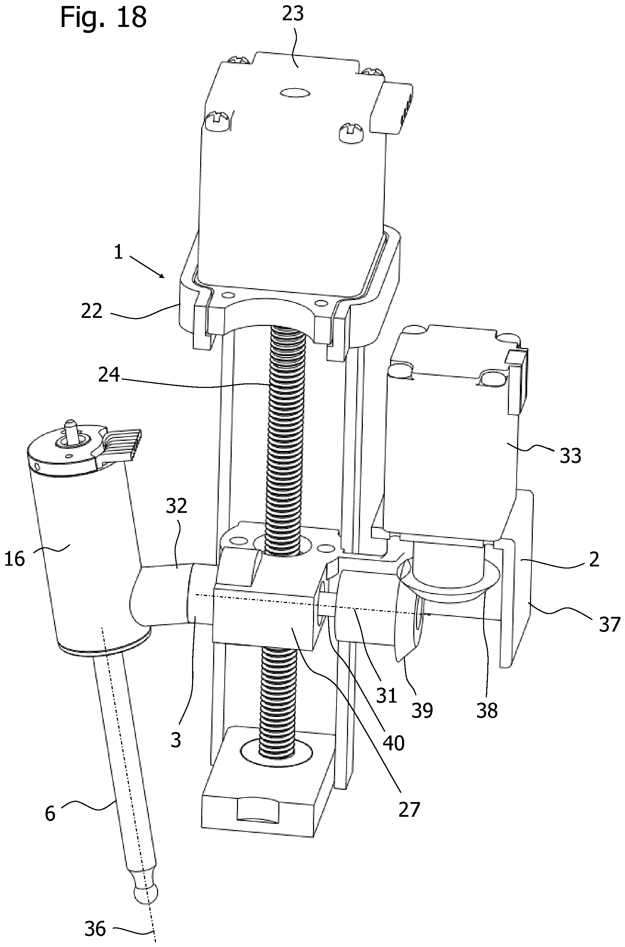

Fig. 18 zeigt eine Detail einer Resektionsvorrichtung 10, bei welcher das Gehäuse 22 sowie das drehbare Verbindungselement 1, welches drehbar um das Basiselement 5 angeordnet ist, teilweise weggelassen ist. Die Resektionsvorrichtung 10 umfasst ein verschiebbares Verbindungselement 2, wobei das verschiebbare Verbindungselement 2 eine Linearbewegung entlang der Drehachse 11 ausführen kann. Die Drehachse 11 ist in dieser Darstellung nicht sichtbar, sie verläuft parallel zu der Mittenachse eines Spindelelements 24, welches Bestandteil des verschiebbaren Verbindungselements 2 ist. Die Resektionsvorrichtung 10 umfasst weiters ein Verbindungselement 3, wobei das Verbindungselement 3 über ein Drehgelenk 32 mit einem der drehbaren oder verschiebbaren Verbindungselemente 1,2 verbunden ist. Das Verbindungselement 3 ist als eine Bearbeitungsvorrichtung zur Durchführung der Knochenresektion ausgebildet. Das Drehgelenk 32 enthält eine Drehgelenksdrehachse 31, wobei das Verbindungselement 3 um die Drehgelenksdrehachse 31 drehbar ist, wobei die Drehgelenksdrehachse 31 nach diesem Ausführungsbeispiel in einem Neigungswinkel zur Drehachse 11 angeordnet ist, der im Bereich von 60 Grad bis einschliesslich 90 Grad liegen kann. Das Verbindungselement 3 enthält einen Endeffektor 6 zur Knochenresektion. Das Verbindungselement 3 kann eine Einheit mit dem Endeffektor 6 ausbilden. Der Endeffektor 6 kann auch an das Verbindungselement 3 gekoppelt werden. Beispielsweise können nach Bedarf unterschiedliche Endeffektoren 6 am Verbindungselement 3 angebracht werden.

Der Endeffektor 6 enthält das Werkzeug 26, insbesondere ein Knochenresektionswerkzeug, bei dem es sich beispielsweise um eine Fräsvorrichtung oder einen Sägeelement handeln kann. Der Endeffektor 6 kann eine Endeffektordrehachse 36 enthalten, um welche eine Rotation des Werkzeugs 26 erfolgen kann. Die Endeffektordrehachse 36 kann mit der Längsachse des Endeffektors 6 zusammenfallen. Zudem kann der Endeffektor einen Endeffektorantrieb 16 aufweisen.

Das drehbare Verbindungselement 1, das verschiebbare Verbindungselement 2 und das Verbindungselement 3 können mittels entsprechenden Verbindungselementantrieben 13, 23, 33 unabhängig voneinander antreibbar sein. Insbesondere kann das drehbare Verbindungselement 1 durch einen ersten, nicht dargestellten Verbindungselementantrieb 13 angetrieben werden, der am nur teilweise dargestellten Gehäuse 22 angebracht ist. Das verschiebbare Verbindungselement 2 wird durch einen zweiten Verbindungselementantrieb 23 angetrieben. Das Verbindungselement 3 wird durch einen dritten Verbindungselementantrieb 33 angetrieben. Nach dem vorliegenden Ausführungsbeispiel bildet das drehbare Verbindungselement 1 ein Gehäuse 22 aus. Das verschiebbare Verbindungselement 2 ist im Gehäuse 22 angeordnet.

Das verschiebbare Verbindungselement 2 umfasst nach diesem Ausführungsbeispiel den zweiten Verbindungselementantrieb 23, das vom zweiten Verbindungselementantrieb 23 angetriebene Spindelelement 24 sowie ein mit dem Spindelelement 24 verschiebbares Schlittenelement 27. Das Schlittenelement 27 enthält eine Halterung 37 für den dritten Verbindungselementantrieb 33. Nach diesem Ausführungsbeispiel sind das Schlittenelement 27 und die Halterung 37 als ein einziges Bauteil ausgebildet. Es ist aber auch nach einem nicht dargestellten Ausführungsbeispiel möglich, dass die Halterung und das Schlittenelement als zwei oder mehrere Bauteile ausgebildet sind, die aber starr miteinander verbunden sind, beispielsweise mit einer Schraubverbindung, einer Schweissverbindung einem anderen Verbindungsmittel. Für die Funktionsweise des Verbindungselements 3 ist es wichtig, dass die Halterung 37 und das Schlittenelement 27 in ihrer Lage zueinander unveränderbar sind.

Der dritte Verbindungselementantrieb 33 ist ausgebildet, ein Antriebskegelrad 38 anzutreiben, welches an einer nicht sichtbaren Antriebswelle des Verbindungselementantriebs 33 befestigt ist. Das Antriebskegelrad 38 steht im Eingriff mit einem Abtriebskegelrad 39, welches auf einer Abtriebswelle 40 angeordnet ist, welche im Schlittenelement 27 drehbar gelagert ist. Die Abtriebswelle 40 kann direkt mit dem Endeffektor 6 verbunden sein und bildet die Drehgelenksdrehachse 31 aus.

Nach jedem der vorhergehenden Ausführungsbeispiele kann eine sterile Abdeckung verwendet werden, um die Resektionsvorrichtung vollständig oder teilweise abzudecken. Die sterile Abdeckung kann auch eine Hülle umfassen oder als Hülle ausgebildet sein. Die Teile, die nicht von der sterilen Abdeckung bedeckt sind, können zum einmaligen Gebrauch bestimmt sein oder sterilisierbar sein.

Die Resektionsvorrichtung 10, 20 nach jedem der Ausführungsbeispiele kann von einer Steuerungseinheit, beispielsweise einem Mikrocontroller, entsprechend einer Vorgabe gesteuert werden. Die Steuerungseinheit kann sich integriert in der Resektionsvorrichtung 10, 20 befinden oder ausserhalb des Arbeitsbereiches. Die Resektionsvorrichtung 10, 20 kann während des Operationsvorgangs in Kommunikation mit der Steuerungseinheit sein, beispielsweise verbunden durch ein Kabel. Zusätzlich kann während des Operationsvorgangs ein Navigationssystem verwendet werden, welches ebenfalls in Kommunikation mit der Steuerungseinheit befinden kann. Ein Bildschirm kann ebenfalls mit der Steuerungseinheit in Kommunikation sein, auf dem Bildschirm können verschiedene Daten angezeigt werden, beispielsweise die Operationsplanung, die aktuelle Position der Resektionsvorrichtung oder der Fortschritt der Operation.

Die Vorgabe zur Resektion des zumindest einen Knochens der Knochenanordnung wird automatisch auf der Grundlage der Resektionsebene berechnet, die von einem Experten, z. B. durch eine Implantatposition, vorgängig festgelegt werden kann. Die Vorgabe kann eine Steuerung des Pfads umfassen, den das Werkzeug zurücklegt, um eine oder mehrere Schichten an Knochenmaterial vom Knochen abzutragen. Vor der autonomen Resektion kann der vorgeschlagene Pfad überprüft und gegebenenfalls angepasst werden.

Ein möglicher Arbeitsablauf mit der Resektionsvorrichtung kann wie folgt aussehen:
- Planen des Eingriffs in einer Planungssoftware unter Einbeziehung von CT-Scans.
- Befestigen des Befestigungselements 4 an der Knochenanordnung 8 und Koppeln der Resektionsvorrichtung 10, 20 mit Hilfe eines optionalen Positionierungs- und Registrierungssystems.
- Durchführen der geplanten Knochenresektion oder Knochenresektionen. Beispielsweise kann die Knochenanordnung 8 eine Mehrzahl von Knochen beinhalten, beispielsweise einen Femur 18 und eine Tibia 28 umfassen. Die Resektion kann an zumindest einem Knochen der Knochenanordnung 8, an einer Mehrzahl der Knochen der Knochenanordnung 8, insbesondere an jedem der Knochen der Knochenanordnung 8 durchgeführt werden.

Optional kann die Spannung von Bändern oder Sehnen ermittelt werden. Bei Bedarf kann ein Anpassungsschnitt durchgeführt werden.

Optional kann ein Tracking-System zur Überwachung des gesamten Verfahrens zum Einsatz kommen.

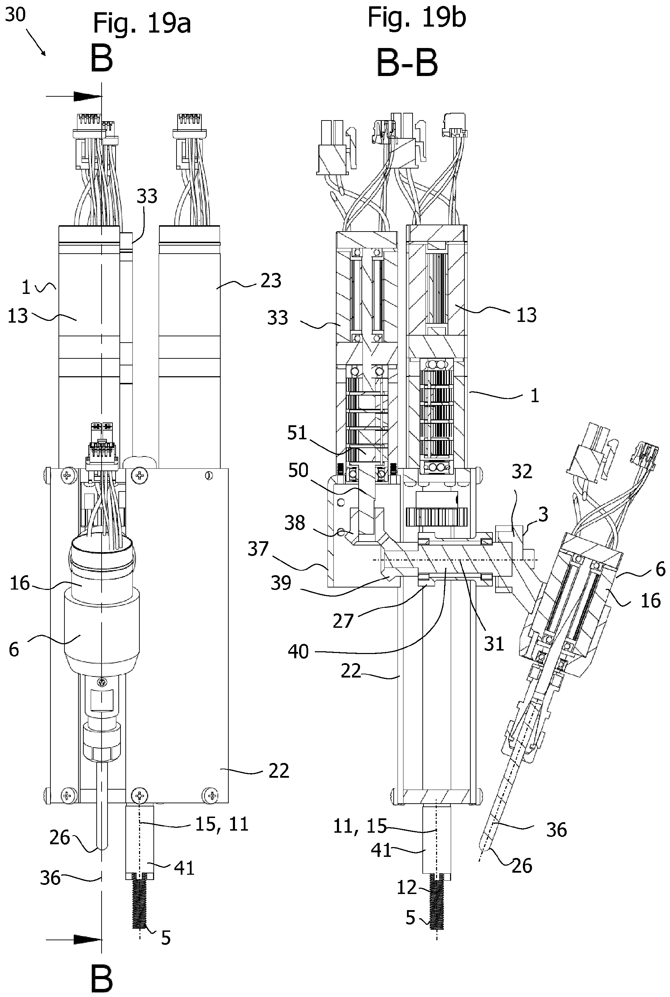

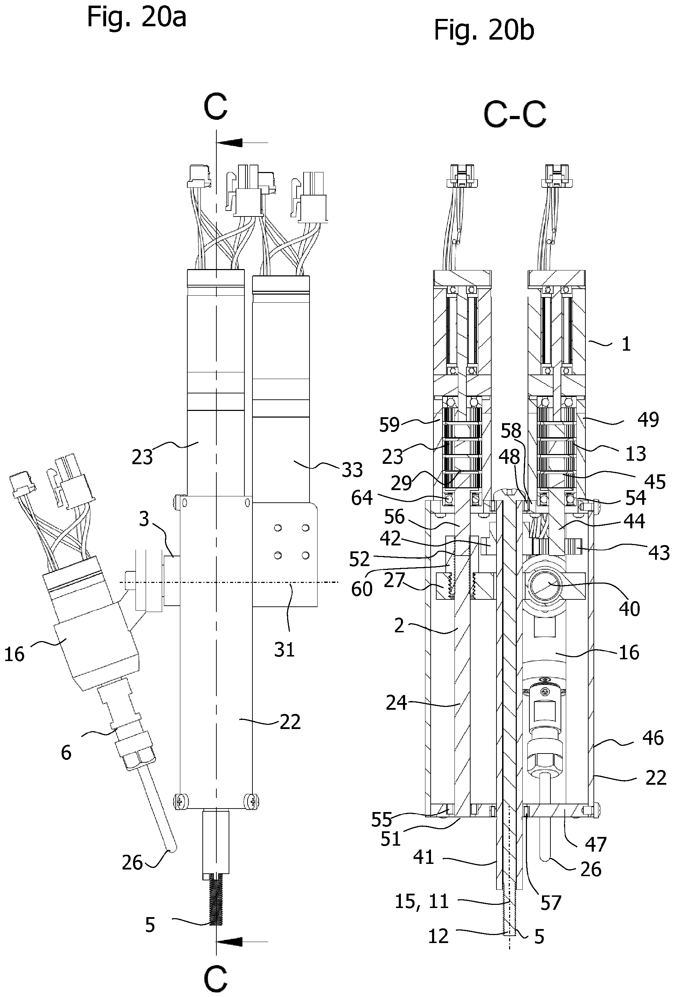

Fig. 19a zeigt eine Ansicht einer Resektionsvorrichtung 30 gemäss eines dritten Ausführungsbeispiels von vorne. Fig. 19b zeigt einen Schnitt durch die Resektionsvorrichtung 30 gemäss Fig. 19a entlang der durch die Schnittlinie B-B repräsentierten Schnittebene. Fig. 20a zeigt eine Seitenansicht der Resektionsvorrichtung 30. Fig. 20b zeigt einen Schnitt durch die Resektionsvorrichtung 30 gemäss Fig. 20a entlang der durch die Schnittlinie C-C repräsentierten Schnittebene. Die Resektionsvorrichtung 30 ist ausgebildet, um an einer Knochenanordnung 8 befestigt zu werden. Für Bauelemente, deren Funktion den früheren Ausführungsbeispielen entspricht, wurden dieselben Bezugszeichen verwendet. Die Resektionsvorrichtung 30 zur Resektion zumindest eines Knochens der Knochenanordnung 8 ohne Benutzerinteraktion umfasst ein Befestigungselement 4, wobei das Befestigungselement 4 dazu ausgebildet ist, starr an der Knochenanordnung 8 befestigt zu werden. Die Knochenanordnung 8 und das Befestigungselement 4 sind in der vorliegenden Darstellung der Einfachheit halber weggelassen, siehe hierzu beispielsweise Fig. 1.

Die Resektionsvorrichtung 30 umfasst ferner ein Basiselement 5, wobei das Befestigungselement das Basiselement 5 enthält oder das Befestigungselement 4 mit dem Basiselement 5 derart koppelbar ist, dass das Basiselement 5 eine starre Verbindung mit dem Befestigungselement 4 ausbildet. Das Basiselement 5 enthält eine Basiselementlängsachse 15. Die Resektionsvorrichtung 30 umfasst weiters ein drehbares Verbindungselement 1, welches drehbar um das Basiselement 5 angeordnet ist, wobei das drehbare Verbindungselement 1 drehbar um die Basiselementlängsachse 15 angeordnet ist, sodass die Basiselementlängsachse 15 eine Drehachse 11 für das drehbare Verbindungselement 1 ausbildet.

Nach dem vorliegenden Ausführungsbeispiel umfasst das Basiselement 5 ein in Fig. 20b im Schnitt gezeigtes Hülsenelement 41, welches mit einem Kernelement 12 des Basiselements 5 fest verbunden ist. Das Hülsenelement 41 enthält ein Abtriebszahnrad 41, welches ebenfalls fest mit dem Hülsenelement 41 verbunden ist. Das Hülsenelement 41 ist über das Abtriebszahnrad 42 mit einem Antriebszahnrad 43 in Eingriff. Das Antriebszahnrad 43 ist mit einer Antriebswelle 44 drehfest verbunden. Das Antriebszahnrad 43 wird vom ersten Verbindungselementantrieb 13 angetrieben. Nach dem vorliegenden Ausführungsbeispiel wird das drehbare Verbindungselement 1 durch das Antriebszahnrad 43 sowie den ersten Verbindungselementantrieb 13 ausgebildet. Der erste Verbindungselementantrieb 13 kann insbesondere die Antriebswelle 44 sowie den Antriebsmotor 45 umfassen. Der Antriebsmotor 45 ist im vorliegenden Beispiel als ein Elektromotor ausgebildet. Wenn das Antriebszahnrad 43 über die Antriebswelle 44 vom Antriebsmotor 45 angetrieben wird, erfolgt eine Drehbewegung des drehbaren Verbindungselements 1 um die Drehachse 11.

Nach dem vorliegenden Ausführungsbeispiel enthält das drehbare Verbindungselement 1 ein Gehäuse 22. Der erste Verbindungselementantrieb 13 befindet sich zumindest teilweise im Gehäuse 22. Das

Gehäuse 22 enthält nach dem vorliegenden Ausführungsbeispiel ein Gehäusemantelelement 46, ein Gehäusebodenelement 47 und ein Gehäuseabdeckelement 48, siehe insbesondere Fig. 20b. Das Gehäuse 22 ist relativ zum Hülsenelement 41 drehbar. Das Gehäuse 22 ist somit derart ausgebildet, dass es bei einer Betätigung des ersten Verbindungselementantriebs 13 eine Drehung um die Drehachse 11 des Basiselements 5 ausführt. Nach dem vorliegenden Ausführungsbeispiel ist das Gehäuse 22 somit Bestandteil des drehbaren Verbindungselements 1. Nach dem vorliegenden Ausführungsbeispiel enthalten das Gehäusebodenelement 47 und das Gehäuseabdeckelement 48 je ein Lagerelement 57, 58. Das Gehäusebodenelement 47 ist mittels des Lagerelements 57 drehbar in Bezug auf das Basiselement 5 gelagert. Nach dem vorliegenden Ausführungsbeispiel befindet sich das Lagerelement 57 auf dem Hülsenelement 41. Das Gehäuseabdeckelement 48 ist mittels des Lagerelements 58 drehbar Bezug auf das Basiselement 5 gelagert. Nach dem vorliegenden Ausführungsbeispiel befindet sich das Lagerelement 58 auf dem Hülsenelement 41.

Der erste Verbindungselementantrieb 13 enthält nach diesem Ausführungsbeispiel ein Antriebsgehäuse 49 für den Antriebsmotor 45. Das Antriebsgehäuse 49 ist ebenfalls mit dem Gehäuse 22 verbunden. Insbesondere kann das Antriebsgehäuse 49 eine Drehbewegung des Gehäuses 22 bei Betätigung des ersten Verbindungselementantriebs 13 mitmachen. Die Antriebswelle 44 ist mittels des Antriebswellenlagerelements 54 drehbar im Antriebsgehäuse 49 gelagert.

Wenn der Antriebsmotor 45 in Betrieb genommen wird, wird die Antriebswelle 44 in eine Drehbewegung versetzt. Die Antriebswelle 44 setzt das Antriebszahnrad 43 in Bewegung. Das Antriebszahnrad 43 ist mit dem Abtriebszahnrad 42 im Eingriff. Das Abtriebszahnrad ist fest mit dem Hülsenelement 41 verbunden, was zur Folge hat, dass das Antriebszahnrad nach der Art eines Planetengetriebes eine Umlaufbewegung um das Abtriebszahnrad 42 ausführt. Diese Umlaufbewegung wird auf das Gehäuse 22 übertragen. Das Gehäuse 22 dreht sich somit mit dem ersten Verbindungselementantrieb 13 um die Drehachse 11. Nach dem vorliegenden Ausführungsbeispiel enthält das Gehäuse 22 das verschiebbare Verbindungselement 2. Somit ist das Gehäuse 22 mit dem ersten Verbindungselementantrieb 13 um die Drehachse 11 drehbar, wobei das verschiebbare Verbindungselement 2 diese Drehbewegung ebenfalls ausführt. Mit anderen Worten ist das verschiebbare Verbindungselement 2 über das Gehäuse 22 mit dem drehbaren Verbindungselement 1 gekoppelt.

Die Drehung des drehbaren Verbindungselements 1 kann im Uhrzeigersinn oder gegen den Uhrzeigersinn erfolgen. Die Drehrichtung des drehbaren Verbindungselements 1 kann geändert werden. Insbesondere kann die Drehung der Antriebswelle 44 im Uhrzeigersinn oder gegen den Uhrzeigersinn erfolgen, wenn der Antriebsmotor 45 in beiden Drehrichtungen betrieben werden kann.

Die Resektionsvorrichtung 30 umfasst weiters ein verschiebbares Verbindungselement 2, wobei das verschiebbare Verbindungselement 2 eine lineare Bewegung parallel zu der Drehachse 11 relativ zum

Basiselement 5 ausführen kann. Nach dem vorliegenden Ausführungsbeispiel ist das verschiebbare Verbindungselement 2 zumindest teilweise im Gehäuse 22 aufgenommen. Das verschiebbare Verbindungselement 2 umfasst nach diesem Ausführungsbeispiel den zweiten Verbindungselementantrieb 23, das vom zweiten Verbindungselementantrieb 23 angetriebene Spindelelement 24 sowie ein mit dem Spindelelement 24 verschiebbares Schlittenelement 27. Der zweite Verbindungselementantrieb 23 umfasst einen Spindelelementantriebsmotor 29, der als ein Elektromotor ausgebildet sein kann sowie eine Antriebswelle 56.

Nach dem vorliegenden Ausführungsbeispiel weist das Spindelelement 24 ein erstes Spindelelementende 51 auf, welches im Gehäusebodenelement 47 des Gehäuses 22 drehbar gelagert ist. Hierzu ist nach dem vorliegenden Ausführungsbeispiel ein Lagerelement 55 im Gehäusebodenelement 47 vorgesehen.

Das Spindelelement 24 weist ein zweites Spindelelementende 52 auf, welches über ein Koppelungselement 60 mit dem zweiten Verbindungselementantrieb 23 koppelbar ist. Der zweite Verbindungselementantrieb 23 kann insbesondere eine Antriebswelle 56 und einen Spindelelementantriebsmotor 29 umfassen. Der zweite Verbindungselementantrieb 23 enthält nach diesem Ausführungsbeispiel ein Antriebsgehäuse 59 für den Antriebsmotor 29. Das Antriebsgehäuse 49 ist ebenfalls mit dem Gehäuse 22 verbunden. Insbesondere kann das Antriebsgehäuse 59 eine Drehbewegung des Gehäuses 22 bei Betätigung des ersten Verbindungselementantriebs 13 mitmachen. Die Antriebswelle 56 ist mittels des Antriebswellenlagerelements 64 drehbar im Antriebsgehäuse 59 gelagert.

Die Drehung des Spindelelements 24 kann im Uhrzeigersinn oder gegen den Uhrzeigersinn erfolgen. Die Drehrichtung des Spindelelements 24 kann geändert werden. Insbesondere kann die Drehung der Antriebswelle 56 im Uhrzeigersinn oder gegen den Uhrzeigersinn erfolgen, wenn der Antriebsmotor 29 in beiden Drehrichtungen betrieben werden kann.

Die Resektionsvorrichtung 30 umfasst weiters ein Verbindungselement 3, wobei das Verbindungselement 3 über ein Drehgelenk 32 mit einem der drehbaren oder verschiebbaren Verbindungselemente 1,2 verbunden ist. Das Verbindungselement 3 ist als eine Bearbeitungsvorrichtung zur Durchführung der Knochenresektion ausgebildet. Das Drehgelenk 32 enthält eine Drehgelenksdrehachse 31, wobei das Verbindungselement 3 um die Drehgelenksdrehachse 31 drehbar ist, wobei die Drehgelenksdrehachse 31 nach diesem Ausführungsbeispiel senkrecht zur Drehachse 11 angeordnet ist. Das Verbindungselement 3 enthält einen Endeffektor 6 zur Knochenresektion. Das Verbindungselement 3 kann eine Einheit mit dem Endeffektor 6 ausbilden. Der Endeffektor 6 kann auch an das Verbindungselement 3 gekoppelt werden. Beispielsweise können nach Bedarf unterschiedliche Endeffektoren 6 am Verbindungselement 3 angebracht werden. Selbstverständlich kann nach einem nicht dargestellten Ausführungsbeispiel das mit dem Bezugszeichen 2 versehene Verbindungselement als das drehbare Verbindungselement ausgebildet sein und das mit dem Bezugszeichen 1 versehene Verbindungselement als das verschiebbare Verbindungselement ausgebildet sein.

Der Endeffektor 6 enthält das Werkzeug 26, insbesondere ein Knochenresektionswerkzeug, bei dem es sich beispielsweise um eine Fräsvorrichtung oder einen Sägeelement handeln kann. Der Endeffektor 6 kann eine Endeffektordrehachse 36 enthalten, um welche eine Rotation des Werkzeugs 26 erfolgen kann. Die Endeffektordrehachse 36 kann mit der Längsachse des Endeffektors 6 zusammenfallen. Zudem kann der Endeffektor 6 einen Endeffektorantrieb 16 aufweisen.

Das drehbare Verbindungselement 1, das verschiebbare Verbindungselement 2 und das Verbindungselement 3 können mittels entsprechenden Verbindungselementantrieben 13, 23, 33 unabhängig voneinander antreibbar sein. Insbesondere kann das drehbare Verbindungselement 1 durch einen ersten Verbindungselementantrieb 13 angetrieben werden, das verschiebbare Verbindungselement 2 durch einen zweiten Verbindungselementantrieb 23 angetrieben werden und das Verbindungselement 3 durch einen dritten Verbindungselementantrieb 33 angetrieben werden. Insbesondere ist zumindest einer der Verbindungselementantriebe 13, 23, 33 in einem kompakten, vom Gehäuse abnehmbaren Block untergebracht. Nach dem vorliegenden Ausführungsbeispiel bildet das drehbare Verbindungselement 1 ein Gehäuse 22 aus und das verschiebbare Verbindungselement 2 ist am Gehäuse 22 angeordnet. Nach dem vorliegenden Ausführungsbeispiel wird vom drehbaren Verbindungselement 1 und vom verschiebbaren Verbindungselement 2 ein gemeinsames Gehäuse 22 ausgebildet. Mittels des ersten Verbindungselements 1 erfolgt über den ersten Verbindungselementantrieb eine Drehung des Gehäuses 22 und des im Gehäuse 22 drehbar gelagerten verschiebbaren Verbindungselements.

Nach diesem Ausführungsbeispiel sind der erste Verbindungselementantrieb 13 und der zweite Verbindungselementantrieb 23 in oder an einem gemeinsamen Gehäuse 22 angeordnet. Mittels des ersten Verbindungselementantriebs 1 wird eine Drehung des Gehäuses 22 der Resektionsvorrichtung 10 um die Basiselementlängsachse 15 ausgeführt, wobei die Basiselementlängsachse 15 die Drehachse 11 ausbildet. Nach diesem Ausführungsbeispiel umfasst das drehbare Verbindungselement 1 das Gehäuse 22. Das verschiebbare Verbindungselement 2 ist nach diesem Ausführungsbeispiel so am Gehäuse 22 angebracht, dass bei einer Drehung des drehbaren Verbindungselements 1 das verschiebbare Verbindungselement 2 von dieser Drehung mit erfasst wird. Die Translationsbewegung des verschiebbaren Verbindungselements 2 kann zeitgleich oder zeitlich versetzt in Bezug auf die Drehung des drehbaren Verbindungselements 1 erfolgen. Es ist also möglich, dass das drehbare und das verschiebbare Verbindungselement 1, 2 gleichzeitig betätigt werden.

Es ist auch möglich, dass zu einem ersten Zeitpunkt nur eine Drehung des drehbaren Verbindungselements 1 erfolgt und zu einem zweiten Zeitpunkt nur die Verschiebung des verschiebbaren Verbindungselements 2 erfolgt. Der erste Zeitpunkt kann sich somit vom zweiten Zeitpunkt unterscheiden. Der erste und der zweite Zeitpunkt können auch zusammenfallen, wenn der erste Verbindungselementantrieb 13 und der zweite Verbindungselementantrieb 23 gleichzeitig in Betrieb sind. Auch die Drehung des Verbindungselements 3 kann zeitgleich oder zeitlich versetzt in Bezug auf die Drehung drehbaren Verbindungselements 1 oder die Translationsbewegung des verschiebbaren Verbindungselements 2 erfolgen. Es ist auch möglich, dass zu einem ersten Zeitpunkt nur eine Drehung des drehbaren Verbindungselements 1 erfolgt und zu einem zweiten Zeitpunkt nur die Verschiebung des verschiebbaren Verbindungselements 2 erfolgt und zu einem dritten Zeitpunkt nur die Drehung oder Verschiebung des Verbindungselements 3 erfolgt. Der erste Zeitpunkt kann sich somit von zumindest einem der zweiten oder dritten Zeitpunkte unterscheiden. Der erste, der zweite und der dritte Zeitpunkt können auch zusammenfallen, wenn der erste Verbindungselementantrieb 13, der zweite Verbindungselementantrieb 23 und der dritte Verbindungselementantrieb 33 gleichzeitig in Betrieb sind.

Nach dem in Fig. 19b dargestellten Ausführungsbeispiel enthält das Schlittenelement 27 eine Halterung 37 für den dritten Verbindungselementantrieb 33. Die Halterung 37 kann auch ein Gehäuseelement umfassen. Nach diesem Ausführungsbeispiel sind das Schlittenelement 27 und die Halterung 37 als ein einziges Bauteil ausgebildet. Es ist aber auch nach einem nicht dargestellten Ausführungsbeispiel möglich, dass die Halterung 37 und das Schlittenelement 27 als zwei oder mehrere Bauteile ausgebildet sind, die aber starr miteinander verbunden sind, beispielsweise mit einer Schraubverbindung, einer Schweissverbindung einem anderen Verbindungsmittel. Für die Funktionsweise des Verbindungselements 3 ist es wesentlich, dass die Halterung 37 und das Schlittenelement 27 in ihrer Lage zueinander unveränderbar sind.

Der dritte Verbindungselementantrieb 33 ist ausgebildet, ein Antriebskegelrad 38 anzutreiben, welches an einer Antriebswelle 50 des Verbindungselementantriebs 33 befestigt ist. Der Verbindungselementantrieb 33 umfasst einen Antriebsmotor 53, die Antriebswelle 50 sowie das Antriebskegelrad 38. Die Antriebswelle 50 verbindet den Antriebsmotor 53 mit dem Antriebskegelrad 38. Das Antriebskegelrad 38 steht im Eingriff mit einem Abtriebskegelrad 39, welches auf einer Abtriebswelle 40 angeordnet ist, welche im Schlittenelement 27 drehbar gelagert ist. Die Abtriebswelle 40 kann direkt mit dem Endeffektor 6 verbunden sein und enthält die Drehgelenksdrehachse 31.

Gemäss dieses Ausführungsbeispiels verfügt die Resektionsvorrichtung über zumindest 3 Freiheitsgrade (DOF). Der erste DOF umfasst eine Rotation des drehbaren Verbindungselements 1 um die Drehachse 11, welche der Basiselementlängsachse 15 des Basiselements 5 entspricht. Der zweite DOF umfasst eine Translationsbewegung des verschiebbaren Verbindungselements 2 in Richtung der Drehachse 11 des drehbaren Verbindungselements 1 insbesondere parallel zu der Drehachse 11. Der dritte DOF umfasst nach dem vorliegenden Ausführungsbeispiel eine Rotation des Verbindungselements 3 um eine Drehgelenksdrehachse 31. Die Drehgelenksdrehachse kann insbesondere senkrecht zur Drehachse 11 des drehbaren Verbindungselements 1 stehen, sie kann auch in einem Winkel von 60 Grad bis einschliesslich 90 Grad zur Drehachse 11 angeordnet sein, was zeichnerisch nicht dargestellt ist.

Für eine Fachperson ist offensichtlich, dass viele weitere Varianten zusätzlich zu den beschriebenen Systemen oder Verfahrensvarianten möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist. Für die Interpretation der Ansprüche oder der Beschreibung ist die breitest mögliche Lesart der Ansprüche massgeblich. Insbesondere sollen die Begriffe "enthalten" oder "beinhalten" derart interpretiert werden, dass sie sich auf Elemente, Komponenten oder Schritte in einer nicht-ausschliesslichen Bedeutung beziehen, wodurch angedeutet werden soll, dass die Elemente, Komponenten oder Schritte vorhanden sein können oder genutzt werden können, dass sie mit anderen Elementen, Komponenten oder Schritten kombiniert werden können, die nicht explizit erwähnt sind. Wenn die Ansprüche sich auf ein Element oder eine Komponente aus einer Gruppe beziehen, die aus A, B, C bis N Elementen oder Komponenten bestehen kann, soll diese Formulierung derart interpretiert werden, dass nur ein einziges Element dieser Gruppe erforderlich ist, und nicht eine Kombination von A und N, B und N oder irgendeiner anderen Kombination von zwei oder mehr Elementen oder Komponenten dieser Gruppe.

## Patentansprüche

1. Eine Resektionsvorrichtung (10, 30) zur Resektion zumindest eines Knochens einer Knochenanordnung (8) ohne Benutzerinteraktion, umfassend:
- ein Befestigungselement (4), wobei das Befestigungselement (4) dazu ausgebildet ist, starr an der Knochenanordnung (8) befestigt zu werden, und
- ein Basiselement (5), wobei das Befestigungselement (4) das Basiselement (5) enthält oder das Befestigungselement (4) mit dem Basiselement (5) derart koppelbar ist, dass das Basiselement (5) eine starre Verbindung mit dem Befestigungselement (4) ausbildet, wobei das Basiselement (5) eine Basiselementlängsachse (15) enthält, und
- ein drehbares Verbindungselement (1), welches drehbar um das Basiselement (5) angeordnet ist, wobei das drehbare Verbindungselement (1) drehbar um die Basiselementlängsachse (15) angeordnet ist, sodass die Basiselementlängsachse (15) eine Drehachse (11) für das drehbare Verbindungselement (1) ausbildet, wobei das drehbare Verbindungselement (1) einen ersten Verbindungselementantrieb (13) enthält, und
- ein verschiebbares Verbindungselement (2), wobei das verschiebbare Verbindungselement (2) einen zweiten Verbindungselementantrieb (23) enthält, wobei mittels des verschiebbaren Verbindungselements (2) eine Linearbewegung entlang oder parallel zu der Basiselementlängsachse (15) relativ zum Basiselement (5) ausführbar ist, und
- ein Verbindungselement (3), wobei das Verbindungselement (3) einen dritten Verbindungselementantrieb (33) enthält, wobei das Verbindungselement (3) über ein Drehgelenk (32) mit einem der drehbaren oder verschiebbaren Verbindungselemente (1,2) verbunden ist, wobei das Drehgelenk (32) eine Drehgelenksdrehachse (31) enthält, wobei das Verbindungselement (3) um die Drehgelenksdrehachse (31) drehbar ist,
- wobei das Verbindungselement (3) einen Endeffektor (6) zur Knochenresektion enthält.

2. Die Resektionsvorrichtung nach Anspruch 1, wobei die Drehgelenksdrehachse (31) in einem Winkel von 60 Grad bis einschliesslich 90 Grad zu zur Drehachse (11) angeordnet ist.

3. Eine Resektionsvorrichtung (20) zur Resektion zumindest eines Knochens einer Knochenanordnung (8) ohne Benutzerinteraktion, umfassend:
- ein Befestigungselement (4), wobei das Befestigungselement (4) dazu ausgebildet ist, starr an der Knochenanordnung (8) befestigt zu werden,
- ein Basiselement (5), wobei das Befestigungselement (4) das Basiselement (5) enthält oder das Befestigungselement (4) mit dem Basiselement (5) derart koppelbar ist, dass das Basiselement (5) eine starre Verbindung mit dem Befestigungselement (4) ausbildet, wobei das Basiselement (5) eine Basiselementlängsachse (15) enthält, und
- ein drehbares Verbindungselement (1), welches drehbar um das Basiselement (5) angeordnet ist, wobei das drehbare Verbindungselement (1) drehbar um die Basiselementlängsachse (15) angeordnet ist, sodass die Basiselementlängsachse (15) eine Drehachse (11) für das drehbare Verbindungselement (1) ausbildet, wobei das drehbare Verbindungselement (1) einen ersten Verbindungselementantrieb (13) enthält, und
- ein verschiebbares Verbindungselement (2), wobei mittels des verschiebbaren Verbindungselements (2) eine Linearbewegung entlang der Basiselementlängsachse (15) relativ zum Basiselement (5) ausführbar ist, wobei das verschiebbare Verbindungselement (2) einen zweiten Verbindungselementantrieb (23) enthält, und
- ein Verbindungselement (3), wobei das Verbindungselement (3) einen dritten Verbindungselementantrieb (33) enthält, wobei das Verbindungselement (3) über ein Führungsschienenelement (34) mit einem der drehbaren oder verschiebbaren Verbindungselemente (1, 2) verbunden ist, wobei das Führungsschienenelement (34) eine Schienenelementachse (35) enthält, wobei das Verbindungselement (3) entlang der Schienenelementachse (35) verschiebbar ist,
- wobei das Verbindungselement (3) einen Endeffektor (6) zur Knochenresektion enthält.

4. Die Resektionsvorrichtung (20) nach Anspruch 3, wobei die Schienenelementachse (35) in einem Winkel von 60 Grad bis einschliesslich 90 Grad zur Drehachse (11) angeordnet ist.

5. Die Resektionsvorrichtung (10, 20) nach einem der vorhergehenden Ansprüche, wobei das Befestigungselement (4) ein Verbindungsstück (7) zur Ausbildung einer starren Verbindung einer Mehrzahl von Knochen der Knochenanordnung (8) enthält.

6. Die Resektionsvorrichtung nach Anspruch 5, wobei das Verbindungsstück (7) ein Befestigungsmittel (17) zur Befestigung am Befestigungselement (4) oder an der Knochenanordnung (8) enthält.

7. Die Resektionsvorrichtung nach Anspruch 6, wobei das Befestigungsmittel (17) zumindest ein Befestigungselement aus der Gruppe bestehend aus Stiftelementen, Schraubelementen oder Klammerelementen umfasst.

8. Die Resektionsvorrichtung nach einem der vorhergehenden Ansprüche, wobei ein Schutzelement (21) für Weichteile vorgesehen ist.

9. Die Resektionsvorrichtung nach einem der vorhergehenden Ansprüche, wobei das drehbare Verbindungselement (1), das verschiebbare Verbindungselement (2) und das Verbindungselement (3) mittels entsprechenden Verbindungselementantrieben (13, 23, 33) unabhängig voneinander antreibbar sind.

10. Die Resektionsvorrichtung nach Anspruch 9, wobei zumindest einer der Verbindungselementantriebe (13, 23, 33) in einem kompakten, abnehmbaren Block untergebracht ist.

11. Die Resektionsvorrichtung nach einem der vorhergehenden Ansprüche, wobei das Befestigungselement (4) mehrere Positionen zur Befestigung des Basiselements (5) enthält.

12. Die Resektionsvorrichtung nach Anspruch 11, wobei die Positionen mittels eines einstellbaren Kopplungselements einstellbar sind.

13. Die Resektionsvorrichtung nach einem der vorhergehenden Ansprüche, wobei das Befestigungselement (4) als ein Befestigungsmittel aus der Gruppe bestehend aus einem intramedullären Stab, einem modifizierten intramedullären Stab, einem Knochennagel, einer Knochenklammer, einer Knochenschraube oder einer mit Stiftelementen befestigten Halterung ausgebildet ist.

14. Die Resektionsvorrichtung nach einem der vorhergehenden Ansprüche, wobei der Endeffektor (6) ein Werkzeug (26) aus der Gruppe bestehend aus einer Fräsvorrichtung, einer Sägevorrichtung oder einer Bohrvorrichtung, umfasst.

15. Die Resektionsvorrichtung nach Anspruch 14, wobei das Werkzeug (26) einen Kugelkopffräser enthält.
